Europäisches Patentamt

European Patent Office

Office européen des brevets

(19)

(11) **EP 1 619 203 A1**

(12) **EUROPEAN PATENT APPLICATION**
published in accordance with Art. 158(3) EPC

(43) Date of publication:
**25.01.2006 Bulletin 2006/04**

(51) Int Cl.:
*C07K 14/52* (1995.01)      *C12N 15/09* (1990.01)
*A61K 38/22* (1995.01)      *A61P 5/38* (2000.01)
*A61P 29/00* (2000.01)      *A61P 35/00* (2000.01)
*A61P 37/06* (2000.01)      *A61P 37/08* (2000.01)

(21) Application number: **04730110.6**

(22) Date of filing: **28.04.2004**

(86) International application number:
**PCT/JP2004/006212**

(87) International publication number:
**WO 2004/096851 (11.11.2004 Gazette 2004/46)**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **28.04.2003 JP 2003124452**

(71) Applicant: **Galpharma Co., Ltd.
Takamatsu-shi,
Kagawa 761-0301 (JP)**

(72) Inventors:
• **HIRASHIMA, Mitsuomi
Takamatsu-shi, Kagawa 761-8071 (JP)**

• **NISHI, Nozomu
Kita-gun, Kagawa 761-0703 (JP)**
• **YAMAUCHI, Akira
Takamatsu-shi, Kagawa 761-0113 (JP)**
• **YOSHIDA, Naoko
Takamatsu-shi, Kagawa 760-0080 (JP)**
• **SEKI, Masako
Kumamoto-shi, Kumamoto 862-0959 (JP)**

(74) Representative: **von Kreisler Selting Werner
Patentanwälte
P.O. Box 10 22 41
50462 Köln (DE)**

(54) **GALECTIN 9-INDUCING FACTOR**

(57)     It has been disclosed that Galectin 9, which is a physiologically active substance acting as a lectin, is expressed in various cells and a correlationship is observed between the expression level of galectin 9 and the metastatic ability of tumors. Therefore, it is presumed that galectin 9 would relate to various physiological phenomena. Thus, a substance allowing the control of galectin 9 production and release is expected as exerting an activity of inducing antitumor and/or anti-inflammatory actions, etc. Therefore, it is required to reveal the same. It has been found that a factor inducing the production and release of galectin 9, "galectin 9-inducing factor", is contained in a certain solubilized tumor cell membrane fraction. This factor can be obtained as a concanavalin A-adsorbed fraction and as a concentrated active fraction by fractionation with an ion exchange column packed with Resource Q@, a hydroxyapatite column, etc. Assay reagents, drugs, assays, etc. can be developed by using the galectin 9-inducing activity of this factor.

EP 1 619 203 A1

SEQUENCE LISTING

<110>  GALPHARMA Co., Ltd.

<120>  Galectin 9-inducing factor

<130>  GL-04PCT

<150>  JP 2003-124452
<151>  2003-04-28

<160>  6

<170>  PatentIn version 3.1

<210>  1
<211>  23
<212>  DNA
<213>  Artificial Sequence

<220>
<223>  Oligonucleotide to act as a primer for PCR

<400>  1
caggcaccca tggctcaaac tac                                          23


<210>  2
<211>  22
<212>  DNA
<213>  Artificial Sequence

<220>
<223>  Oligonucleotide to act as a primer for PCR

<400>  2
tatcagactc ggtaacgggg gt                                           22


<210>  3
<211>  24
<212>  DNA
<213>  Artificial Sequence

<220>
<223>  Oligonucleotide to act as a primer for PCR

<400>  3
cgtcaatggc tctgtgcagc tgtc                                         24


<210>  4
<211>  24
<212>  DNA
<213>  Artificial Sequence

<220>
<223>  Oligonucleotide to act as a primer for PCR

<400>  4
agatccacac tgagaagctc tggc                                         24

<210> 5
<211> 24
<212> DNA
<213> Artificial Sequence

<220>
<223> Oligonucleotide to act as a primer for PCR

<400> 5
ggtcagagtt caaggtgatg gtga                                                    24


<210> 6
<211> 24
<212> DNA
<213> Artificial Sequence

<220>
<223> Oligonucleotide to act as a primer for PCR

<400> 6
gcctgatatc atgatggact tgga                                                    24


**Description**

FIELD OF THE INVENTION

The present invention relates to factors which are active in induction of galectin 9, i.e., galectin 9-inducers. Particularly, the present invention relates to mammal galectin 9-inducers including human galectin 9-inducers. The present invention also relates to application techniques of said galectin 9-inducers.

BACKGROUND OF THE INVENTION

The inventor and et al. have succeeded in the cloning of a human T cell-derived eosinophilic chemotactic factor and found (Non-Patent Document 2) that it was ecalectin, a variant of human galectin-9 reported by Tureci et al. (Non-Patent Document 1). In addition, t he inventor and et al. have demonstrated that ecalectin and galectin-9 are identical substances and that there are 3 types for human galectin-9, i.e., short, medium, and long types, depending on the length of the link peptide (Non-Patent Document 7).

[Non-Patent Document 1] Tureci O. et al., J Biol Chem, Mar. 7, 1997, 272(10): 6416-22
[Non-Patent Document 2] Matsumoto R. et al., J Biol Chem, 1998, 273: 16976-84

SUMMARY OF THE INVENTION

It has been disclosed that Galectin 9, which is a physiologically active substance acting as a lectin, is expressed in tissue mast cells, eosinophils, macrophages, T cells, B cells, fibroblasts, endothelial cells, various tumor cells and other cells, and a correlationship is observed between the expression level of galectin 9 and the metastatic ability of tumors. Therefore, it is presumed that galectin 9 would relate to various physiological phenomena. Thus, a substance allowing the control of galectin 9 production and release is expected as exerting an activity of inducing antitumor and/or anti-inflammatory actions, etc. Therefore, it is required to reveal the same. Galectin 9 is thought to be involved in physiological actions which are important for a variety of living bodies. For instance, galectin 9 can induce apoptosis in activated T lymphocytes. Therefore, it is expected that the control of galectin 9 production and release would allow regulation of diverse physiological phenomena and biologically active things. Factors allowing regulation of intracellular galectin levels, and galectin 9 expression and release, are expected as useful and advantageous tools for pharmaceuticals.

The present inventors have made an extensive study. As a result, they have succeeded in finding that a certain soluble cell membrane fraction (hereinafter referred to as "mf") contains a factor inducing the production and release of galectin 9 (often abbreviated herein to "Gal-9"). In particular, it has been successfully found that solubilized tumor cell membrane fractions contain the factors that induce the production and release of Gal-9. It has also been successfully found that said mf contains the factors that incite the cellular infiltration of Gal-9 producing cells at sites administered and the production and release of Gal-9 in or from such cells. Said factor is designated herein as "galectin 9-inducer" or "galectin 9-inducing factor". In light of biological activities and/or physiological activities of said factors, utilization of said factors allows induction of antitumor and anti-inflammatory actions.

The present invention provides the following:

[1] A human galectin 9-inducer which has an identifiable biological activity existing in a soluble cell membrane fraction derived by solubilization of insoluble cell lysates of human lymphoid B cell lines, BALL-1 cells (B lymphoma cells), wherein the biological activity of said galectin 9-inducer can be identified by at least a property selected from the group consisting of:

(1) galectin 9-inducing activity,
(2) ability to incite inhibition or suppression of tumor cell growth and/or tumor rejection in an in vivo test wherein Meth-A sarcoma cells are used as tumor cells to be targeted,
(3) antitumor activity,
(4) ability to induce the natural killer activity of peripheral blood mononuclear cells (MNC) in an in vitro test,
(5) up-regulation of galectin 9 mRNA expression in a test wherein peripheral blood mononuclear cells are used,
(6) significant elevation in the cytoplasmic expression of galectin 9 proteins in a test wherein peripheral blood mononuclear cells are used,
(7) the formation of recognizable granulation tissue composed of eosinophils and mononuclear cells, accompanied with few neutrophils, at a site injected with said galectin 9-inducer when histopathologically examined,
(8) the induction of a large number of observable mast cells at connective tissues over or underneath the cutaneous muscle layer of said galectin 9 inducer-injected site,

(9) the induction of observable regions with infiltrated inflammatory cells (predominant eosinophils and a few mast cells), at the periphery of tumors or being located within tumor tissues, when the tumors or the peripheral areas of tumors are histopathologically examined,

(10) the formation of observable tumor cells showing pyknotic changes when the tumors or the peripheral areas of tumors are histopathologically examined, and

(11) the occurrence of observable metachromatic mast cell accumulation in regions at the periphery of tumors or within tumor tissues when the tumors or the peripheral areas of tumors are histopathologically examined.

[2] The galectin 9-inducer according to the above [1] , wherein the starting cells used as sources are radiated lymphoid B cell lines BALL-1 cells.

[3] The galectin 9-inducer according to the above [1] or [2] , which exists in a soluble cell membrane fraction derived by solubilization including homogenization of BALL-1 cells with a detergent in the presence of a protease inhibitor.

[4] The galectin 9-inducer according to any of the above [1] to [3] , which can be purified and/or concentrated from said B lymphoma cell-derived soluble cell membrane fraction by a treatment selected from the group consisting of concanavalin A column chromatography, ion exchange column chromatography, hydroxyapatite column chromatography, and other column chromatographic techniques.

[5] A galectin 9-inducing reagent for intracellular induction of galectin 9, which comprises an effective amount of the galectin 9-inducer according to any of the above [1] to [4].

[6] A method for intracellular induction of galectin 9, which comprises contacting a cell with an effective amount of the galectin 9-inducer according to any of the above [1] to [4] .

[7] A pharmaceutical drug which comprises an effective amount of the galectin 9-inducer according to any of the above [1] to [4] .

[8] The pharmaceutical drug according to the above [7] , which is selected from antineoplastic drugs, anti-inflammatory drugs, antiallergic drugs, immunosuppressants, drugs for auto-immune diseases, and adrenal cortical steroid hormone alternatives.

[9] The galectin 9-inducer according to any of the above [1] to [4] , which is derived from a human-derived source.

ADVANTAGEOUS PROFILES OF THE INVENTION

The galectin 9-inducing factors (galectin 9-inducers) are successfully identified and purified herein, thereby leading to applications of said purified galectin 9-inducers in order to develop pharmaceutical products and to accelerate research and development of physiological phenomena and biological actions associated with galectin 9. In particular, galectin 9-inducers are contained in a soluble cell membrane fraction, a concanavalin A-adsorbed fraction from said fraction, and a concentrated active fraction derived by fractionation with a Resource Q@ ion exchange column, a hydroxyapatite column, etc. Administration of said inducer leads to occurrence of biological activities including antitumor activity and the activity of enhancing natural killer activity and others. Therefore, it will be possible to develop assay reagents, pharmaceutical products, assays and the like based on adaptations of said galectin 9-inducing activity.

The above objects and other objects, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

BRIEF DESCRIPTION OF THE DRAWINGS

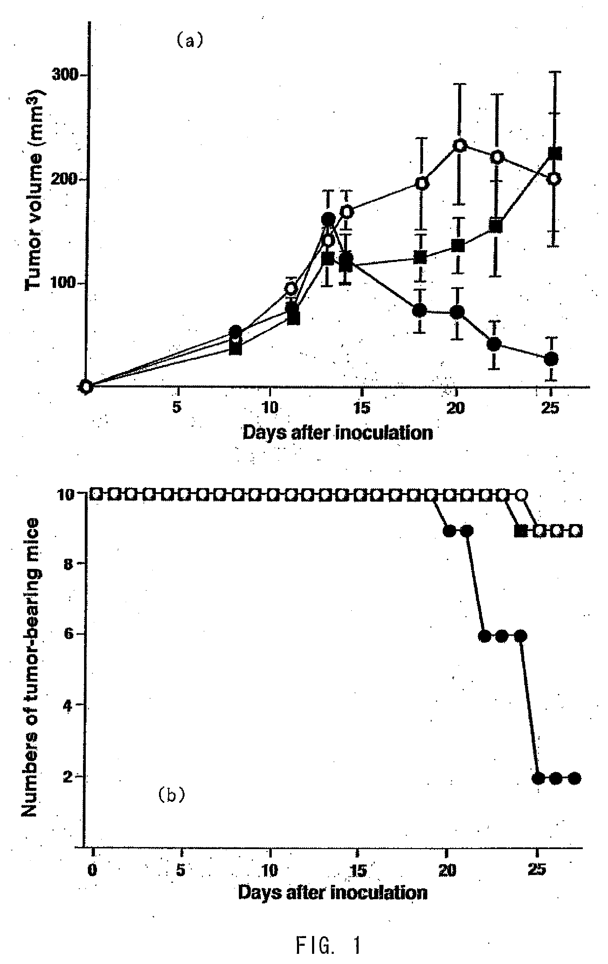

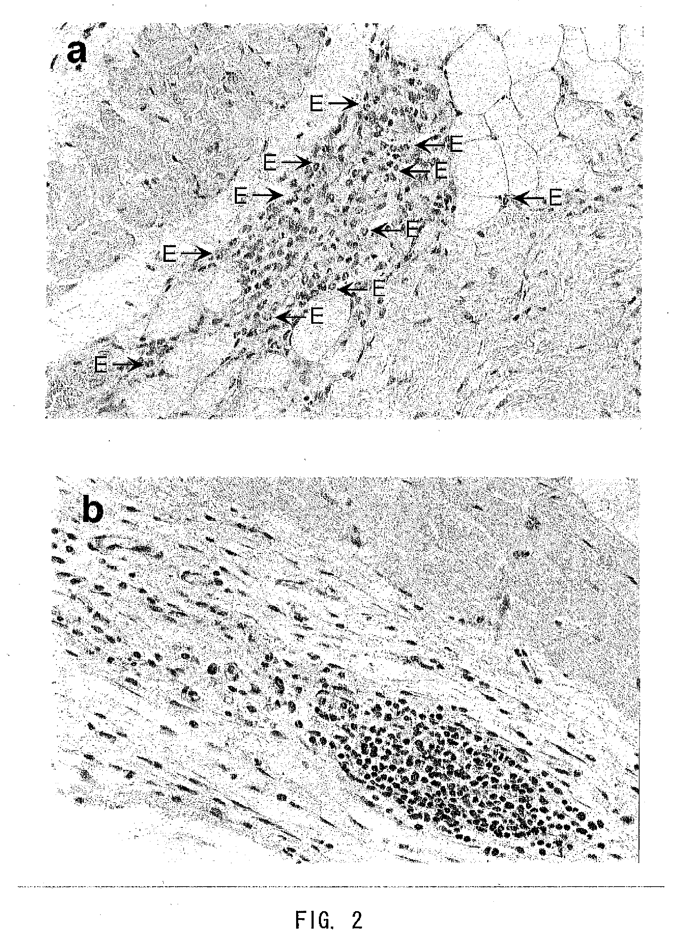

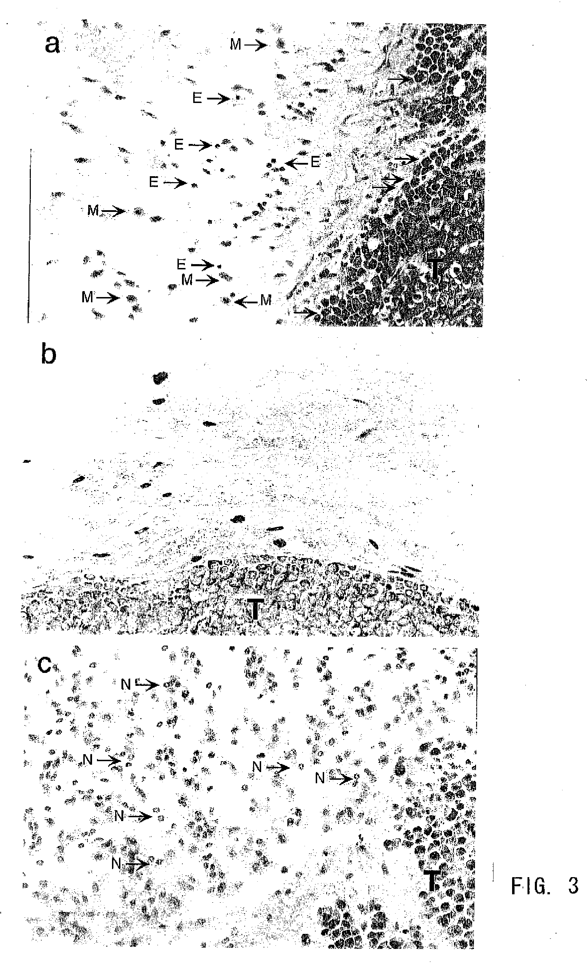

Fig. 1 shows antitumor test results of BALL-mf. (a): The inhibitory efficacy of BALL-mf on tumor growth is shown. •: Tumor weight in BALL-mf treated animals. ■ : Tumor weight in Daudi-mf treated animals. ○: Tumor weight in PBS treated animals. On day 18 post-treatment, the tumor inhibitory efficacy of BALL-mf was significant (n=10, p<0.05). (b): Test results for tumor rejection in sarcoma, Meth A-bearing mice. •: Number of BALL-mf treated animals wherein tumor rejection was induced. ■ : Number of Daudi-mf treated animals wherein tumor rejection was induced. ○: Number of PBS treated animals wherein tumor rejection was induced. Chi-square ($\chi^2$) analysis (n=10, p=0.0006).

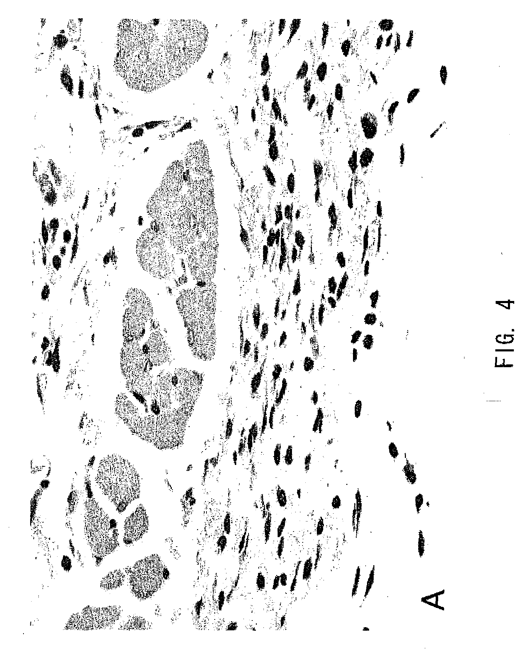

Fig. 2 is a set of photomicrographs showing histopathologically examined sections taken from tissues of Meth A-bearing mice. On day 27 after treatment of Meth A-bearing mice, the skin section was excised, and subjected to fixation followed by staining with Giemsa stain. (a): Treated with BALL-mf. (b): Treated with Daudi-mf. Arrows with E indicate eosinophils.

Fig. 3 is a set of photomicrographs showing histopathologically examined sections taken from tissues of Meth A-bearing mice treated with BALL-mf. On day 27 post-treatment with BALL-mf, the tumor site was excised, and subjected to fixation followed by staining with Giemsa stain (a) or toluidine blue stain (b). Similarly, the tumor site of mice treated with Daudi-mf was excised, and subjected to fixation followed by staining with Giemsa stain (c). Arrows with E indicate eosinophils, those with M do mast cells, and those with N do neutrophils, respectively. Meth A cells with pyknosis are indicated by arrows alone.

Fig. 4 is a photomicrograph showing a histopathologically examined section taken from the tissue of mice treated with BALL-mf. At 24 hours after intracutaneous injection of BALL-mf into the dorsal trunk (local site) of mice, the tissue was taken out, and stained with Giemsa stain. It was observed that lymphocytes and tissue mast cells were infiltrated together with remarkable eosinophils. When infiltrated cells were examined, predominant mast cells and eosinophils (arrow) were observed together with few lymphocytes and macrophages.

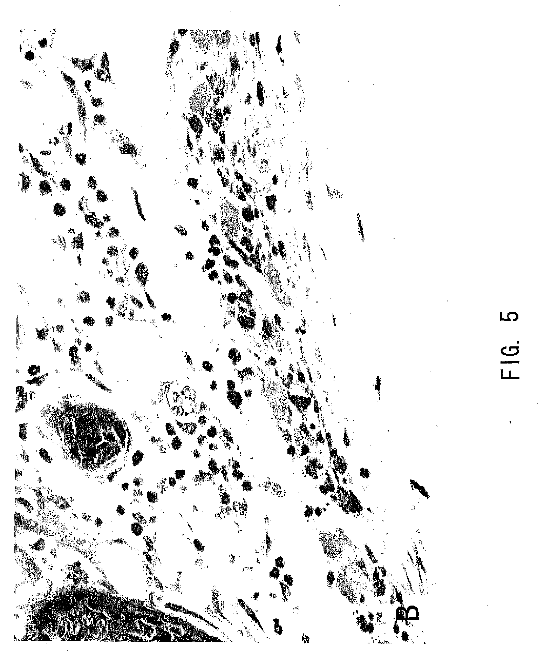

Fig. 5 is a photomicrograph showing a histopathologically examined section taken from the tissue of mice treated with Daudi-mf (control). When Daudi-mf was intracutaneously injected into the dorsal site of mice, the infiltration of lymphocytic cells was significant while no infiltration of eosinophils was observed. Neither infiltrated mast cells nor infiltrated eosinophils were observed but the infiltration of lymphocytes was significant.

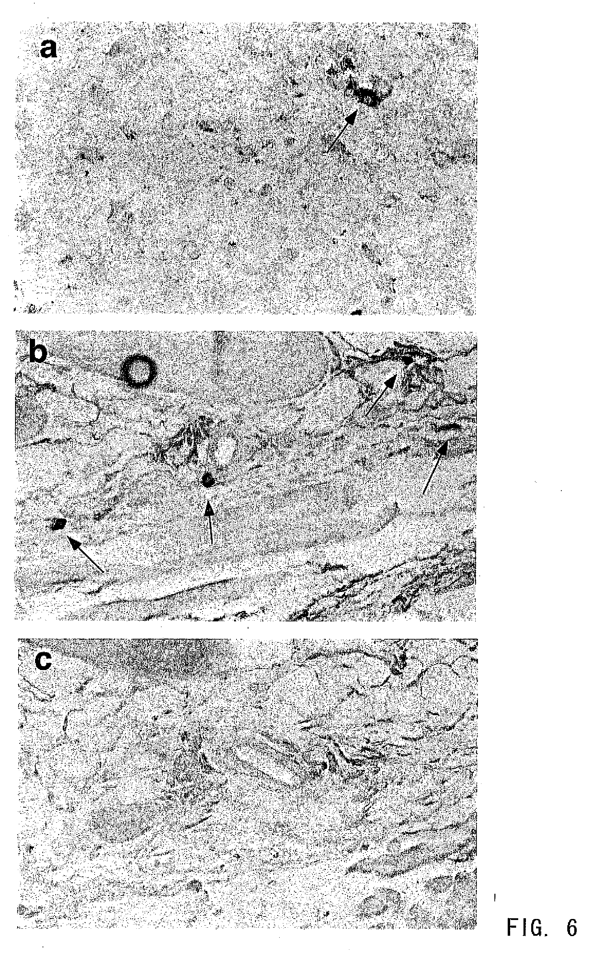

Fig. 6 is a set of photomicrographs showing in situ hybridization to galectin mRNA in infiltrated cells which appeared after injection with BALL-mf. Results: (a) mast cells contained galectin 9 mRNA at a high level, and eosinophils, macrophages and fibroblasts contained galectin 9 mRNA at a slight level. (b) The infiltration of mast cells, which contained galectin 9 mRNA at a high level, was observed in sites just over the panniculus carnosus muscle. In the control group (injection with Daudi-mf), the infiltration of mast cells was not observed at the panniculus carnosus muscle site (c). Galectin 9 was not observed in infiltrated lymphocytes.

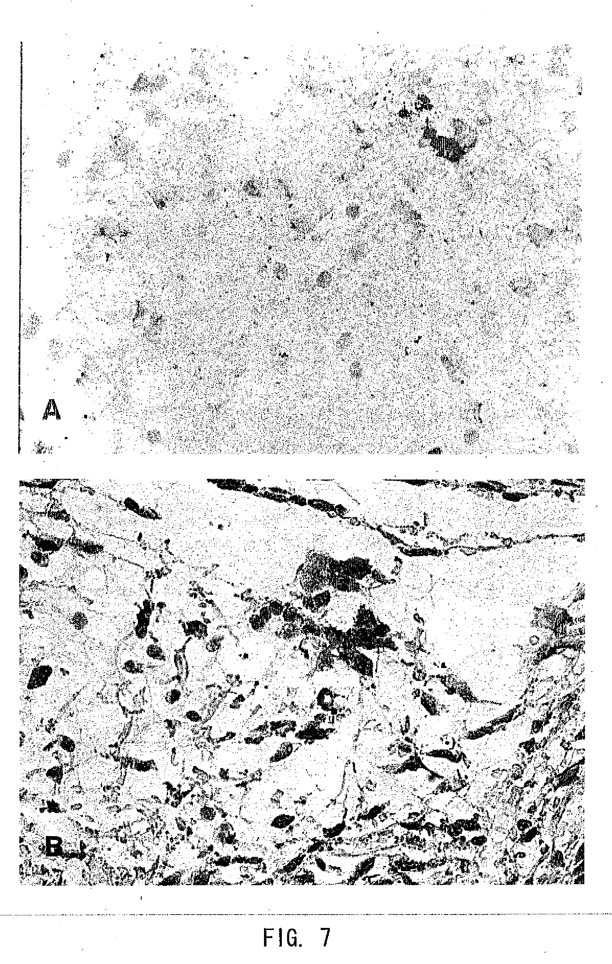

Fig. 7 is a set of photomicrographs showing the in vivo efficacy of galectin 9-inducers. In order to disclose galectin 9-producing cells and/or galectin 9-releasing cells which appeared at sites injected with BALL-mf, each sample was examined by in situ hybridization (A) and immunostaining (B). In the in situ hybridization (A), main galectin 9-producing cells were identified to be mast cells and others included fibroblasts, lymphocytes, eosinophils, and the like, which had galectin 9 genes. In the immunostaining (B), it was found that the aforementioned cells contained galectin 9 in their cytoplasms. These suggest that the BALL-mf stimulation leads to the production and/or release of galectin 9 from such inflammatory cells, thereby resulting in induction of inflammatory responses.

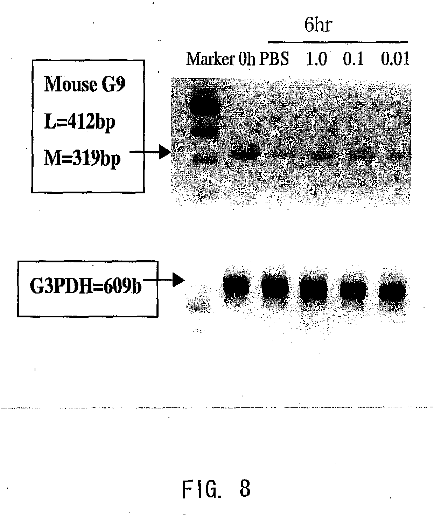

Fig. 8 shows test results for the galectin 9-producing/releasing efficacy of BALL-mf. Mouse peritoneal cells were stimulated with BALL-mf, and subjected to mRNA extraction. The resultant mRNA samples were quantitatively assayed by RT-PCR. As a result, it was found that the expression of galectin 9 mRNA was increased with BALL-mf though it was slight. Further, Western blotting and FACS analysis were conducted. By FACS analysis, it was disclosed that cytoplasmic galectin 9 proteins were decreased in BALL-mf stimulated cells. These results suggest that BALL-mf enhances the production of galectin 9 and it would induce the release of galectin 9 rather than.

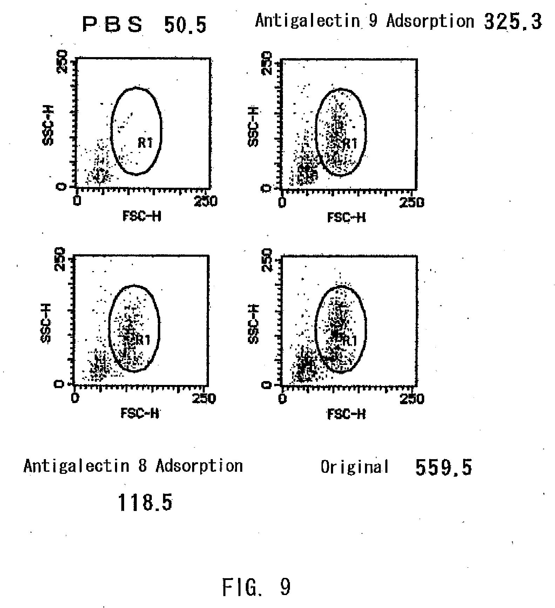

Fig. 9 shows test results for the galectin 9-producing/releasing efficacy of BALL-mf. In order to examine whether or not the release of galectin 9 was induced in BALL-mf stimulated PC culture supernatants, eosinophil chemotactic activity was measured. As a result, it was observed that the eosinophil chemotactic activity was increased, and moreover such an eosinophil chemotactic activity was adsorbed on an anti-galectin 9 antibody (anti-Gal-9 Ab) column. It was found that the release of galectin 9 was increased. The BALL-mf mediated eosinophil chemotactic activity was adsorbed by anti-Gal-9 Ab while not by anti-Gal-8 Ab. Therefore, it is thought to be attributable to an action of galectin 9-inducers. Galectin 9-inducers induce the production of galectin 9 in not only mast cell lineage cells but also eosinophil lineage cells and T cell lineage cells.

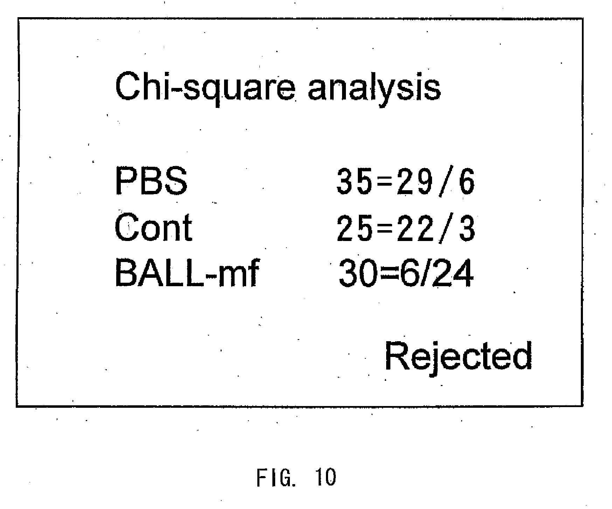

Fig. 10 shows in vivo test results for the antitumor efficacy of BALL-mf. It was found that BALL-mf inhibited or suppressed the successful transplantation and/or growth of Meth-A tumor cells. In chi-square ($\chi^2$) analysis, the inoculated tumor cells were successfully transplanted and grown in 29 animals among a total of 35 control group animals (treated with PBS), and in 22 animals among a total of 25 Daudi-mf treated animals, while the cells were eradicated or nontransplanted in 24 animals among a total of 30 BALL-mf treated animals.

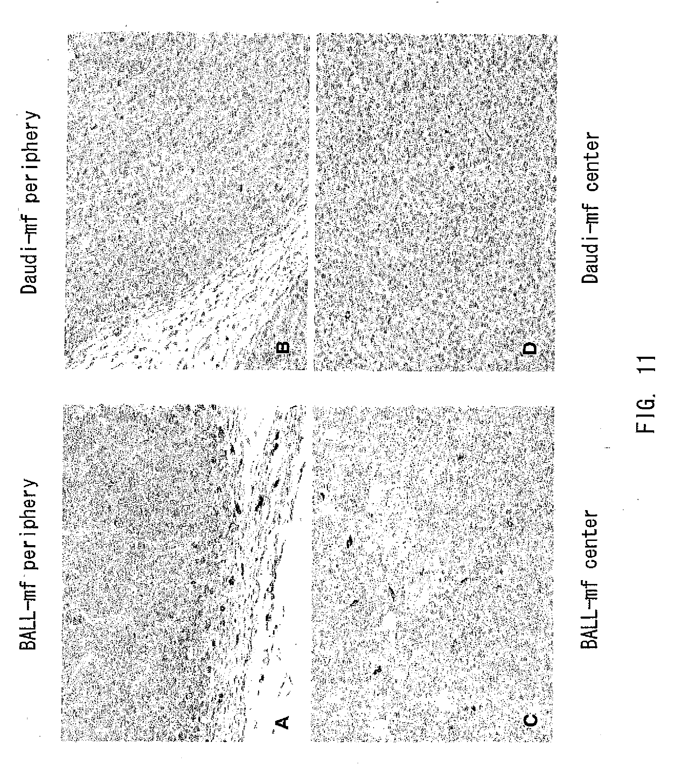

Fig. 11 is a set of photomicrographs showing immunohistochemically analyzed tumor tissue sections. When immunohistochemical staining was conducted with anti-galectin 9 Ab, it was observed in the BALL-mf treated group that the BALL-mf periphery contained infiltrated mast cells having remarkably galectin 8 (A) and mast cells were infiltrated within tumor regions (BALL-mf center). It was also noted that galectin 9 was expressed in tumor cells (A) . In contrast, it was observed in the Daudi-mf treated group that no galectin 9-expressing cells were present and galectin 9 was scarcely expressed in tumor cells (Daudi-mf center).

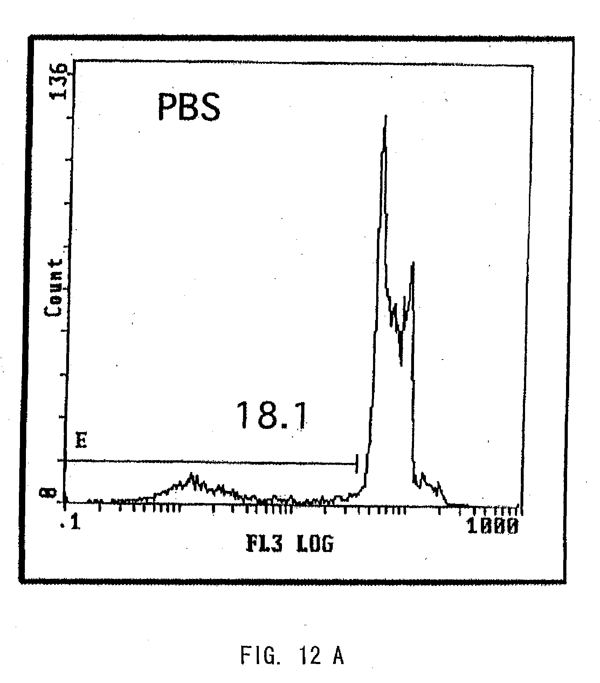

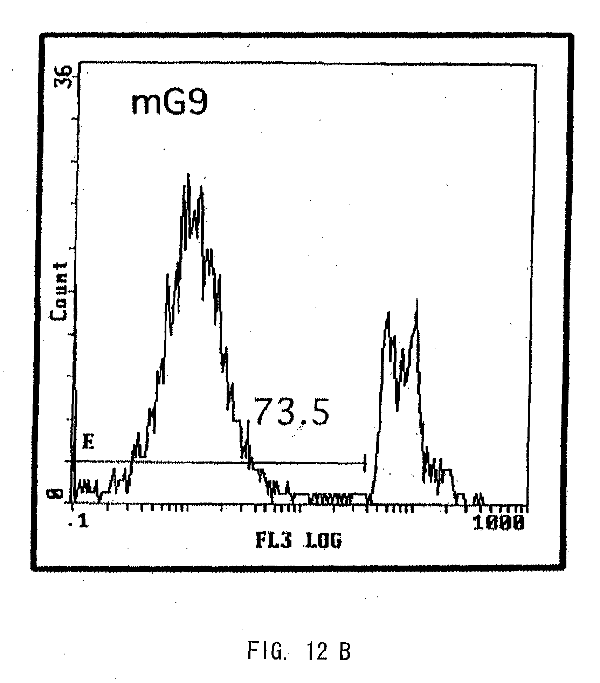

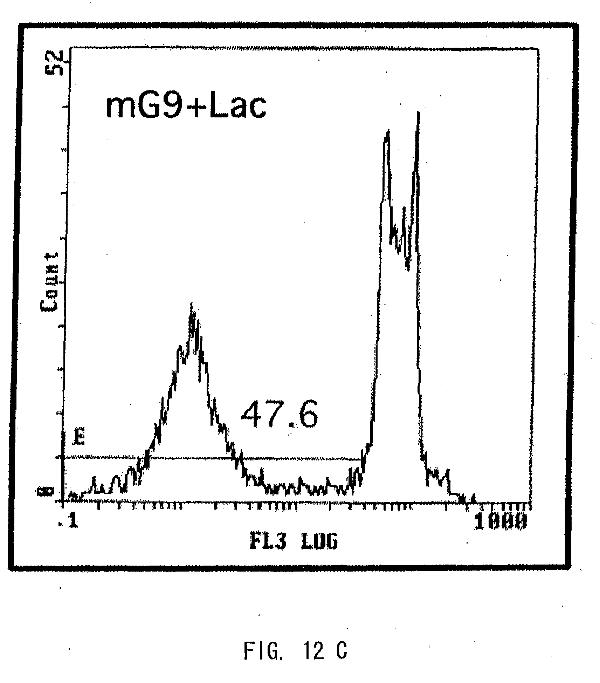

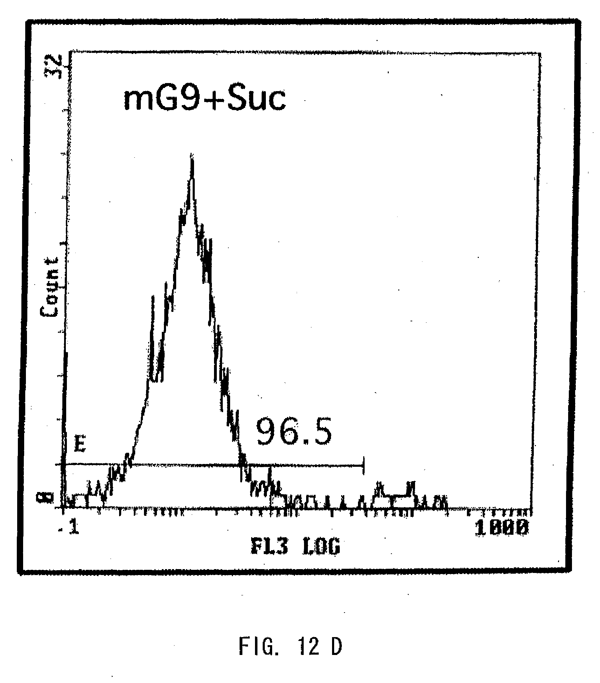

Fig. 12 shows test results for the apoptosis-inducing efficacy of galectin 9 on Meth-A tumor cells. Galectin 9 induces apoptosis in Meth-A cells.

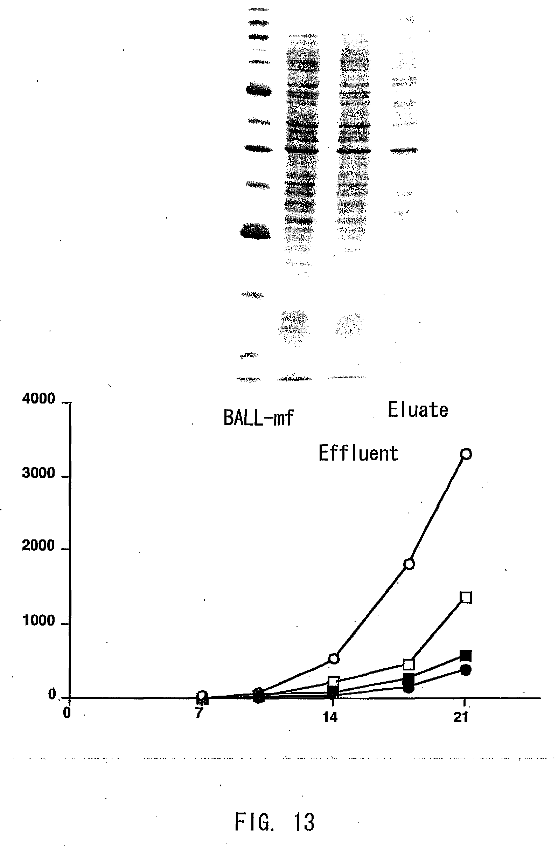

Fig. 13 shows test results for purification of galectin 9-inducers by lentil lectin affinity chromatography and antitumor actions. Unadsorbed and adsorbed fractions were separately obtained by chromatography on a lentil lectin column, and assayed for their galectin 9-inducing activity. As a result, the galectin 9-inducing activity was observed mainly in the adsorbed fractions. In antitumor activity assay experiments, the antitumor efficacy levels of the adsorbed fraction (Eluate) were observed to be comparable to those of Original (BALL-mf). The infiltration levels of eosinophils and mast cells for the adsorbed fraction were similar to those for Original. In the drawing, ○ stands for PBS, □ for Effluent, ■ for Eluate, and • for Original.

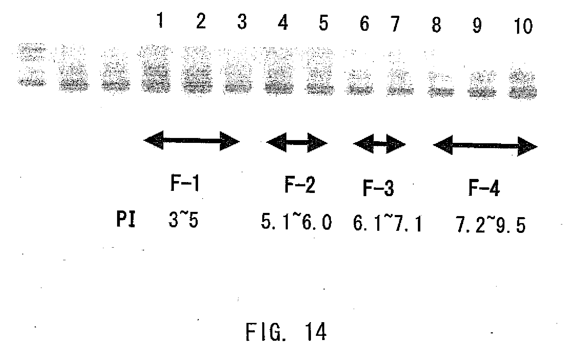

Fig. 14 shows test results for isoelectric fractionation and antitumor efficacy of galectin 9-inducers. RNA samples were collected from peripheral blood mononuclear cells stimulated with fractions resulting from isoelectric focusing (IEF) of lectin column-adsorbed fractions on the Rotofor®, and examined by RT-PCR for galectin 9 expression. The expression of galectin 9 was apparently observed to be enhanced in F-1, F-2, and F-4 with RT-PCR.

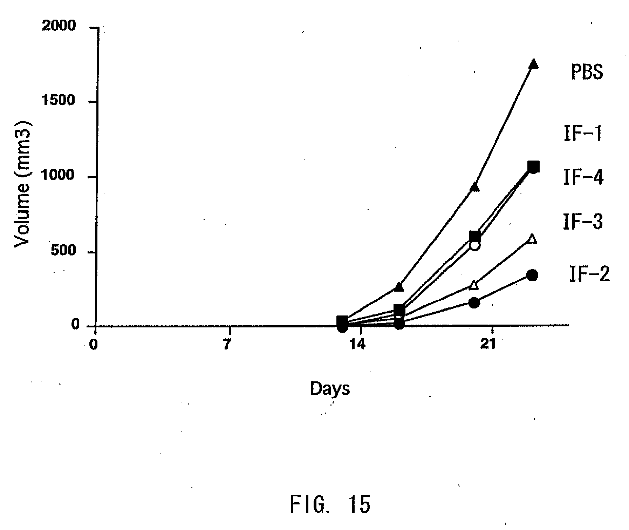

Fig. 15 shows antitumor activity test results of isoelectrically focused fractions as shown in Fig. 14. The intense antitumor activity is induced by F-2 and F-3. The efficacy of F-1 and F-4 is similar to that of PBS, or with increased tumor cell growth. The antitumor activity was observed in the inducers contained in F-2 and F-3. The infiltration of eosinophils and mast cells was observed with tissue staining.

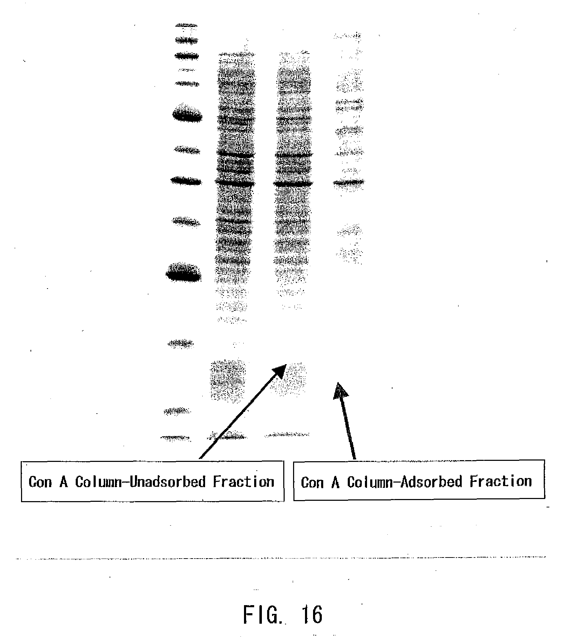

Fig. 16 shows results for purification of BALL-mf by Con A affinity column chromatography. BALL-mf was fractionated on a Con A column to give unadsorbed and adsorbed fractions, which were subjected to SDS-PAGE. As a result, different protein bands were observed.

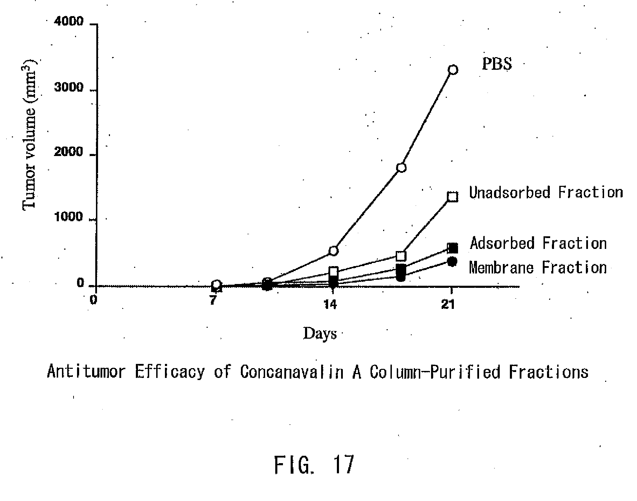

Fig. 17 show antitumor efficacy test results for Con A column-fractinated fractions. The adsorbed fractions exerted more intense antitumor activity. This indicates that the antitumor inducers are glycoproteins having mannose or glucose.

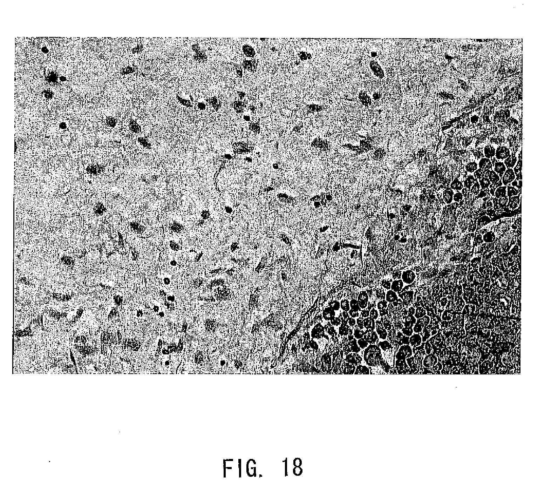

Fig. 18 is a photo showing the resulting tissue in the cytotoxicity examinations of Con A column-adsorbed BALL-mf fractions.

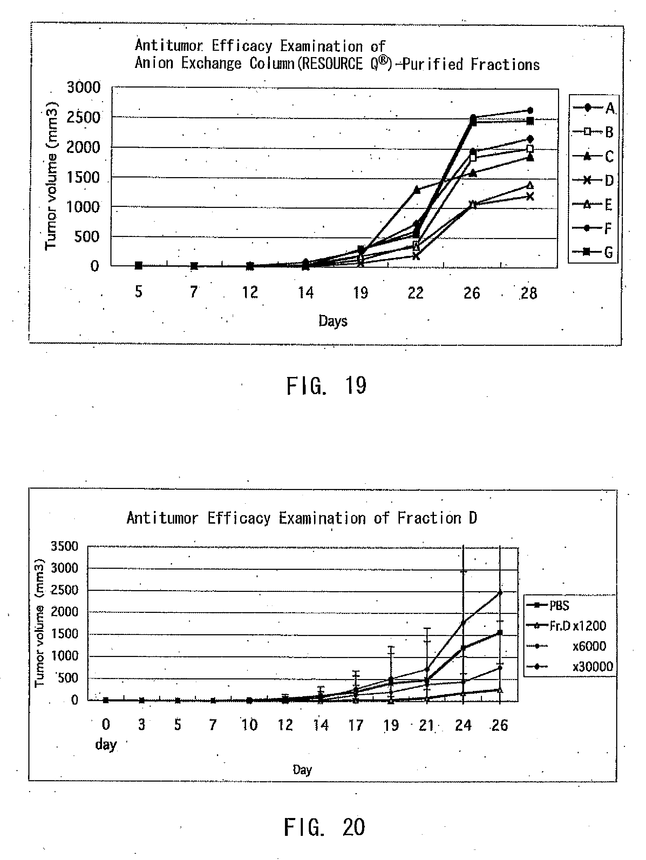

Fig. 19 shows antitumor results for each of the resulting fractions (A to G) from anion exchange column (RESOURCE Q®)-purification of Con A column-adsorbed BALL-mf fractions.

Fig. 20 shows concentration-dependent antitumor results for fraction D from anion exchange column fractionation of Con A column-adsorbed BALL-mf fractions. Data indicate dose-dependent antitumor activity.

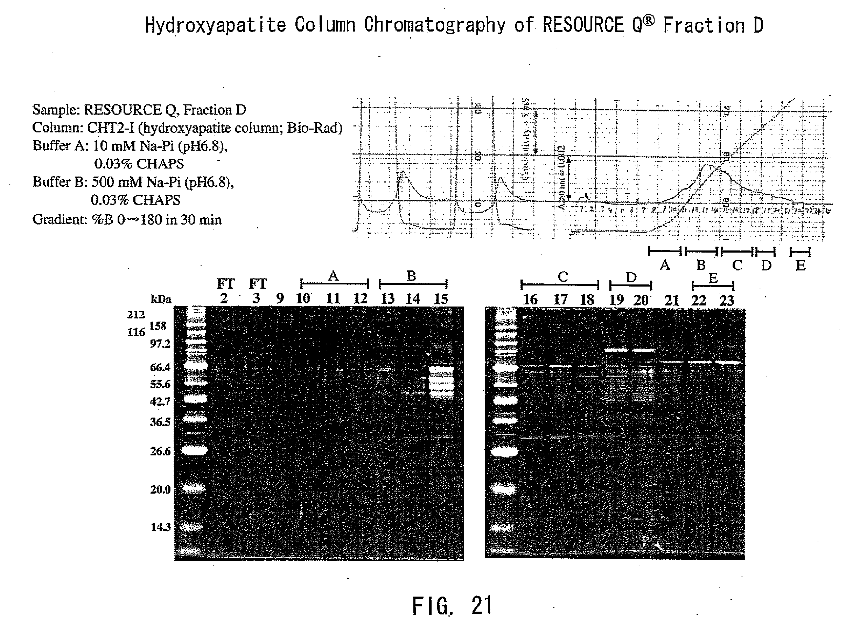

Fig. 21 shows hydroxyapatite column (CHT2-I) fractionation patterns (elution patterns) of anion exchange column (RESOURCE Q®)-purified fraction D and photos for electrophoretic profiles of respective CHT2-I fractions.

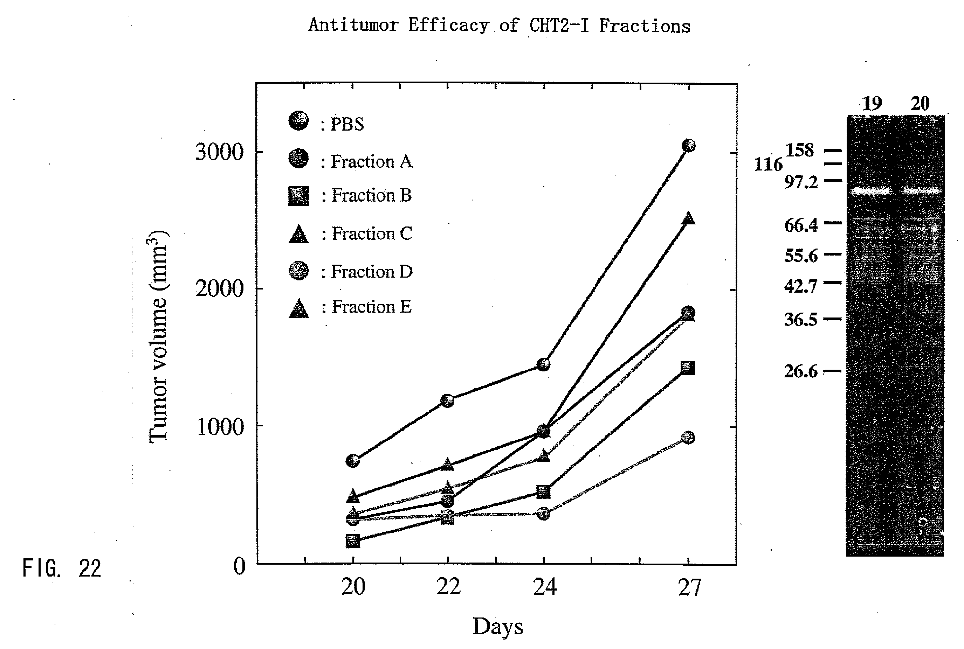

Fig. 22 shows antitumor results for respective hydroxyapatite column (CHT2-I)-purified fractions and a photo for electrophoretic profiles of CHT2-I fraction D.

BEST MODES OF CARRYING OUT THE INVENTION

The galectin 9-producing and/or releasing cells herein include mast cells, eosinophils, macrophages, T cells, B cells, fibroblasts, endothelial cells, various tumor cells, and other cells. Materials or compositions containing said galectin 9-inducer include B cell line-derived mf (for example, human acute lymphoblastoid leukemia (ALL)-derived human cell line: BALL-1 mf, etc.), mf eluate collected by elution of said B cell line-derived mf (e.g., BALL-1 mf) solutes adsorbed on a concanavalin A (Con A) column, Resource Q® ion exchange column mf eluate, hydroxyapatite column eluate fractions, derived therefrom, etc.

Said galectin 9-inducers can be identified by detecting and/or measuring (monitoring) in vitro or in vivo their biological activity (e.g., galectin 9-inducing activity). For instance, the in vitro galectin 9-inducing activity can be assayed through stimulating the aforementioned galectin 9-producing and/or releasing cells with mf, and then quantitatively or qualitatively analyzing Gal-9 mRNA and/or Gal-9 proteins by a technique selected from RT-PCR, Western blotting, flowcytometry, immunohistostaining, ELISA, ELISPOT, RIA, and other techniques. It can be assayed through using the cell culture medium of said cells and quantitatively or qualitatively analyzing Gal-9 proteins by a technique selected from RT-PCR, Western blotting, flowcytometry, immunohistostaining, ELISA, ELISPOT, RIA, and other techniques. The in vivo galectin 9-inducing activity can be assayed through administrating mf to an animal such as mouse, rat, guinea pig, rabbit, and monkey, and then using, as an indicator, infiltration of galectin 9-producing cells and/or enhancement of Gal-9 release. It can be assayed through using a direct or indirect increase in a level of Gal-9 in tumor cells as an indicator. Representatives of said animals include experimental animals. The administration routes include intracutaneous, subcutaneous, intramuscular, intravenous or intra-arterial, and intraperitoneal injections, drinking or eating, etc.

When the galectin 9 is put into action, it is possible to induce aggregation and apoptosis in tumor cells, further to attain antitumor activity, and to induce apoptosis in CD4-positive T cells, thereby allowing the control of allergy and/or autoimmune diseases, and a trial of inhibiting inflammation, attributable to an overreaction of the CD4-positive T cell.

The galectin 9-inducers of the present invention are characterized in that they have ability to induce the expression of galectin 9. Said inducers are characterized in that their presence or expression leads to induction of significant galectin 9 expression. Said inducers exert a variety of physiological and/or biological activities through induction of galectin 9. The galectin 9-inducer of the present invention is isolated from, for example, radiated lymphoid B cell lines, BALL-1 cells (B lymphoma cells). BALL-1 cells for allowing isolation of the inventive galectin 9-inducer are available from American Type Culture Collection (ATCC; Manassas, Virginia, USA); JCRB Cell Bank, Japanese Collection of Research Bioresources (JCRB), National Institute of Health Sciences (NIHS), Ministry of Health, Labor and Welfare, Japan: Health Science Research Bioresources Bank (HSRRB), Japan Health Sciences Foundation, Osaka, Japan). The aforementioned cells are usually cultured in a customary medium for cultivation of human-derived cells, such as 10% fetal calf serum (FCS)-containing RPMI1640 medium. The culture media used herein are not limited to, as long as said cell lines can be grown, but include, for example, liquid nutrient media containing saccharides, amino acids, vitamins, other organic nutrient elements, trace inorganic salts, and others. The cells are grown, then, as required, radiated, further cultured as the case may be, collected (e.g., collected by centrifugation, or other techniques), and disrupted in a buffer (e.g., physical disruption with glass beads, or biochemical technique using sonication, or with enzyme and the like) to give cell-free extracts or lysates. Typically, the galectin 9-inducers of the present invention can be concentrated, isolated and/or purified from soluble cell membrane fractions or supernatants, derived from BALL-1 cells. Said soluble BALL-1 cell membrane fraction can be prepared, for example, by homogenizing BALL-1 cells with a detergent (surface-active agent) in the presence of a protease inhibitor (e.g., phenylmethylsulfonyl fluoride, etc.) to solubilize components, followed by centrifugation to give a supernatant, which is then dialyzed and passed through a 0.2$\mu$m pore size filter. The galectin 9-inducers of the present invention can be purified by a technique alone, or any suitable combination thereof, selected from the group consisting of fractionation techniques according to protein solubility (solvent precipitation utilizing an organic solvent, salting out such as ammonium sulfate precipitation, etc.); dialysis; cation exchange chromatography; anion exchange chromatography; gel filtration; hydrophobic chromatography; and affinity chromatography utilizing a member selected from chelates, dyes, antibodies and other ligands. In an embodiment, the galectin 9-inducer can be purified, in the form of an electrophoretically near single band, through anion exchange chromatography using a member selected from DEAE Sepharose® and other media; affinity chromatography on Blue Sepharose®; and/or high performance liquid chromatography systems using a member selected from Mono Q® HR 5/5 (FPLC® system, Amersham Pharmacia Biotech) and others. In a representative case, the polyacrylamide gel electrophoresis allows acquisitions in the form of a near single band protein product. In a specific embodiment, the galectin 9-inducer can be purified and/or concentrated, from the aforementioned soluble BALL-1 cell membrane fraction (BALL-1 cell lysate) by a treatment selected from the group consisting of concanavalin A (Con A) column chromatography, anion exchange column chromatography, and hydroxyapatite column chromatography. Quantitative protein assay can be conducted with a commercially available protein assay kit, for example by a dye-binding technique. It is also possible to use protein autoanalyzers for such a protein assay.

DNA encoding the galectin 9-inducer of the present invention can be subjected to isolation according to techniques as described herein below. Briefly, after purification of said inducer, its N-terminal amino acid sequence is analyzed (sequenced). In amino acid sequence analysis for said inducer, purified samples are, as required, digested with an enzyme such as lysyl endopeptidase, and V8 protease, then subjected to purification by reverse liquid chromatography and other techniques to give peptide fragments which are analyzed for their amino acid sequence with a protein sequencer. In the sequence analysis, amino acid sequencing can be accomplished using a plurality of peptide fragments. PCR primers are designed on determined amino acid sequences, and PCR is performed using said inducer-producing cell chromosome DNA or cDNA library (may be selected from commercially available products) as a template in combination with PCR primers designed on the amino acid sequence to obtain part of targeted DNA according to the present invention. Upon these steps, human genome data bases (GenBank®, DNA Data Bank of Japan (DDBJ), etc.) can be utilized, for example, via retrieval with a suitable program (e.g., BLAST program, etc.). Further, desired DNA can be obtained by colony hybridization, plaque hybridization, or other techniques, using the resultant DNA fragment as a probe in combination with a gene library obtained by steps of inserting restriction digests of said galectin 9-inducer producing cell chromosome DNA into a member selected from phages, plasmids or other vechicles, followed by transformation of E. coli, or cDNA library. The nucleotide sequence of PCR DNA fragment products is analyzed, and primers for amplifying regions outside of already known DNA are designed on the resulting sequence. Inverse PCR can be performed by steps of digestion of said galectin 9-inducer producing cell chromosome DNA with a suitable restriction enzyme, and self-ligation of the resultant restriction digests to make a circular DNA followed by amplification with the DNA as a temple to give desired DNA (Ochman, H. et al., Genetics, 120: 621-623 (1988); Innis, M. et al. (Ed.), PCR: Application & Protocols, Academic Press, New York (1989)); also, RACE can be done (Rapid Amplification of cDNA Ends, Frohman, M.A. et al. Proc. Natl. Acad. Sci. USA, 85: 8998 (1988); Innis, M.A. et al. (Ed.), PCR Protocols: A guide to methods and applications, pp 28-38, Academic Press, New York (1990), Tohru Komano (Ed.), Seibutsu Kagaku Jikken Ho 47 PCR Jikken Manual, Japan Scientific Society Press (JSSP), Tokyo), etc. Said desired DNA includes genome DNA or cDNA, cloned by methods as aforementioned; besides, the DNA can be chemically synthesized.

The galectin 9-inducers of the present invention have identifiable biological activity wherein said biological activity can be identified using at least a property selected from the group consisting of

(1) galectin 9-inducing activity,

(2) ability to incite inhibition or suppression of tumor cell growth and/or tumor rejection in an in vivo test wherein Meth-A sarcoma cells are used as tumor cells to be targeted,

(3) antitumor activity,

(4) ability to induce the natural killer activity of peripheral blood mononuclear cells (MNC) in an in vitro test,

(5) up-regulation of galectin 9 mRNA expression in a test wherein peripheral blood mononuclear cells are used,

(6) significant elevation in the cytoplasmic expression of galectin 9 proteins in a test wherein peripheral blood mononuclear cells are used,

(7) the formation of recognizable granulation tissue composed of eosinophils and mononuclear cells, accompanied with few neutrophils, at a site injected with said galectin 9-inducer when histopathologically examined,

(8) the induction of a large number of observable mast cells at connective tissues over or underneath the cutaneous muscle layer of said galectin 9 inducer-injected site,

(9) the induction of observable regions with infiltrated inflammatory cells (predominant eosinophils and a few mast cells), at the periphery of tumors or being located within tumor tissues, when the tumors or the peripheral areas of tumors are histopathologically examined,

(10) the formation of observable tumor cells showing pyknotic changes when the tumors or the peripheral areas of tumors are histopathologically examined, and

(11) the occurrence of observable metachromatic mast cell accumulation in regions at the periphery of tumors or within tumor tissues when the tumors or the peripheral areas of tumors are histopathologically examined.

Typically, the galectin 9-inducing activity can be used as an indicator. Galectin 9-inducing activity can be identified by assaying changes in the existing level of galectin 9, changes in galectin 9 activity, changes in galectin 9-expressing activity, changes in the level of galectin 9 mRNA, and the like, not only when the galectin 9-inducer of the present invention is added but also when it is not added. Galectin 9 activity or galectin 9-expressing activity can be assayed, for example, according to assay methods as disclosed in WO 02/37114 A1.

In the present invention, utilization of "gene recombination techniques" allows not only acquisition, isolation, and sequencing of targeted nucleic acids, polynucleotides, proteins, peptides and fragments thereof, but also creation and production of recombinant constructs thereof. Gene recombination techniques (including recombinant DNA techniques) as can be used herein include those known in the art, and can be carried out by the methods described in, for example, J. Sambrook et al., "Molecular Cloning: A Laboratory Manual", Cold Spring Harbor Laboratory Press, Cold Spring Harbor, New York (2nd Edition, 1989 & 3rd Edition, 2001); D. M. Glover et al. ed., "DNA Cloning", 2nd ed., Vol. 1 to 3, (The Practical Approach Series), IRL Press, Oxford University Press (1995); "Methods in Enzymology" series, Academic Press, New York, e.g., R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987); R. Wu ed., "Methods in Enzymology", Vol. 216 (Recombinant DNA, Part G), Academic Press, New York (1992); R. Wu ed., "Methods in Enzymology", Vol. 217 (Recombinant DNA, Part H) & 218 (Recombinant DNA, Part I), Academic Press, New York (1993); P. M. Conn ed., "Methods in Enzymology", Vol. 302 (Green Fluorescent Protein), Academic Press, New York (1999); S. Weissman ed., "Methods in Enzymology", Vol. 303 (cDNA Preparation and Characterization), Academic Press, New York (1999), etc., or by methods described in the references quoted therein or methods substantially equivalent thereto or modified methods thereof, the disclosures of which are incorporated herein by reference.

The term "oligonucleotide(s)" used herein refers to a relatively short single-stranded polynucleotide or double-stranded polynucleotides, or preferably polydeoxynucleotide(s). They can be chemically synthesized by known methods as described in Angew. Chem. Int. Ed. Engl., Vol. 28, pp.716-734 (1989), including phosphotriester, phosphodiester, phosphite, phosphoramidite, phosphonate methods, and the like. It has been typically known that the synthesis can be conveniently carried out on modified solid supports. For example, the synthesis can be carried out using an automated synthesizer and such a synthesizer is commercially available. Examples of the synthesizers as can be used herein are Applied Biosystems 3400 DNA synthesizer (Applied Biosystems), ABI 3900 High-Throughput DNA synthesizer (Applied Biosystems). The oligonucleotide may contain one or more modified nucleotide bases and, for example, it may contain a nucleotide base which does not naturally occur, such as inosine, or a tritylated nucleotide base. In some cases, they may contain one or more nucleotide bases tagged with a marker.

The term "polymerase chain reaction" or "PCR" used herein usually refers to techniques described in H. A. Erlich ed., PCR Technology, Stockton Press, 1989 and other documents. For example, the PCR is an in vitro method for the enzymatic amplification of desired specific nucleotide sequences. In general, the PCR includes repetitive series of cycles wherein a primer elongation synthesis is constructed using two oligonucleotide primers capable of preferentially hybridizing with a template nucleic acid. Typically, the primers used in PCR may include those which are complementary to the internal nucleotide sequence of interest in the template. For example, preferable primer pairs as used herein may be those which are complementary to both ends of said nucleotide sequence to be amplified, or flanking regions adjacent to said nucleotide sequence. The primers include oligonucleotides made up of preferably 5 or more nucleotide bases, more preferably 10 or more nucleotide bases, and still preferably 18 to 25 nucleotide bases.

The PCR reactions can be carried out by methods known in the art or methods substantially equivalent thereto and modified methods thereof. For example, the PCR can be performed according to methods described in R. Saiki, et al., Science, 230: 1350, 1985; R. Saiki, et al., Science, 239: 487, 1988; D. M. Glover et al. ed., "DNA Cloning", 2nd ed., Vol. 1, (The Practical Approach Series), IRL Press, Oxford University Press (1995); M. A. Innis et al. ed., "PCR Protocols: a guide to methods and applications", Academic Press, New York (1990)); M. J. McPherson, P. Quirke and G. R. Taylor (Ed.), PCR: a practical approach, IRL Press, Oxford (1991); M. A. Frohman et al., Proc. Natl. Acad. Sci. USA, 85, 8998-9002 (1988) and other documents, and modified methods or variants thereof. The PCR methods can also be performed using commercially available kits suitable therefor, and can also be carried out according to protocols disclosed by manufacturers or distributors of the kits.

In a representative case, the PCR is performed for example, using a template (e.g., DNA synthesized using mRNA as a template; 1st strand DNA) and primers synthesized according to designs on said gene, in admixture with a 10X reaction buffer (contained in a Taq DNA polymerase kit), dNTPs (deoxyribonucleoside triphosphates; dATP, dGTP, dCTP and dTTP mix), Taq DNA polymerase and deionized distilled water. The mixture is subjected to 25 to 60 cycles of amplification using an automated thermal cycler such as GeneAmp® PCR system 2700 (Applied Biosystems) under general PCR cycle conditions. The number of amplification cycles can be suitably set to an appropriate value depending on purposes. The PCR cycle includes, for example, denaturation at 90 to 95°C for 5 to 100 sec, annealing at 40 to 60°C for 5 to 150 sec and extension at 65 to 75°C for 30 to 300 sec, and preferably denaturation at 94°C for 15 sec, annealing at 58°C for 15 sec and extension at 72°C for 45 sec. For the annealing temperature and reaction time, an appropriate value is suitably selected by experimentation. For the denaturation and extension time, an appropriate value suitably varies according to the strand length of expected PCR products. In general, the annealing reaction time preferably varies depending on the Tm value of primer-template DNA hybrids. The time period of extension is usually set with the aim of getting about 1 min per 1000 bp in strand length, but it may be possible to select a shorter time period in some cases.

Identification of the target nucleic acids (polynucleotides) can be conducted by adaptations of hybridization techniques. The hybridization may be carried out according to methods as disclosed in documents mentioned in the aforementioned "gene recombination techniques", or substantially equivalent methods and modifications thereof. Examples of such hybridization techniques as can be used herein are colony hybridization, plaque hybridization, hybridization translation assay, plus-minus screening, and others. For instance, the hybridization is achieved by transferring a sample containing a nucleic acid such as DNA onto a carrier including a membrane such as a nylon filter, as required, optionally followed by denaturation, fixation, washing, etc., and then reacting the transfers on the carrier (e.g., membrane), with labeled DNA probe fragments which are, as required, optionally denatured in a hybridization buffer. The probe, etc. may be labeled by a radioactive isotope using a commercially available labeling kit, such as the Random Prime DNA Labeling Kit (Boehringer Mannheim), etc. For example, a random priming kit may be used to label the probe DNA with $[\alpha\text{-}^{32}P]$ dCTP (Amersham), etc. and thus provide a probe with radioactivity. The labeling is carried out by known methods in the art. Representatives of labels are digoxigenin (DIG), fluorescent dyes, biotin-avidin systems and others.

The hybridization operations can be ordinarily conducted at about 35°C to about 80°C, more preferably about 50°C to about 65°C, for about 15 min to about 36 hours, more preferably about 1 to about 24 hours, but optimal hybridization conditions may be suitably selected. For example, the hybridization is carried out at about 55°C for about 18 hours. The hybridization buffers can be selected from those customarily used in the art. The denaturation of carriers (e.g., membranes) with transfers includes techniques using an alkali denaturing solution. It is preferable to treat the carrier with a neutralizing solution and a buffer solution after the denaturation. The carrier fixation (e.g., membrane fixation) is usually achieved by baking at about 40°C to about 100 °C, more preferably about 70°C to about 90 °C, for about 15 min to about 24 hours, more preferably about 1 to about 4 hours, but desired fixation conditions may be suitably selected. For example, the fixation is carried out by baking at about 80°C for about 2 hours. The washing of carriers (e.g., membranes) with transfers can be performed with washing solutions customarily used in the art, such as 50mM Tris-HCl buffer, pH8.0, containing 1M NaCl, 1mM EDTA and 0.1% sodium dodecyl sulfate (SDS). The carriers including membranes can be selected from those customarily used in the art. Examples of such carriers include nylon filters.

The alkaline denaturing solution, neutralizing solution and buffer solution can be selected from those conventionally used in the art. The alkaline denaturing solution may include, for example, solutions containing 0.5M NaOH and 1.5M NaCl, etc. The neutralizing solution may include, for example, 0.5M Tris-HCl buffers (pH8.0) containing 1.5M NaCl, etc. The buffer solution may include, for example, 2XSSPE (0.36M NaCl, 20mM $NaH_2PO_4$ and 2mM EDTA), etc. As required, prior to hybridization, it is desired to optionally prehybridize carriers (e.g., membranes) containing transferred DNA and the like, to prevent nonspecific hybridization. For the prehybridization, the sample is dipped, for example, in a solution for prehybridization (50% formamide, 5X Denhardt's solution (0.2% bovine serum albumin and 0.2% polyvinylpyrrolidone), 5 X SSPE, 0.1% SDS, and 100 μg/ml thermally denatured salmon sperm DNA) and the like, and reacted at about 35°C to about 50°C, preferably about 42°C, for about 4 to about 24 hours, preferably about 6 to about 8 hours. These conditions can be determined by those of skill in the art with suitably repeated experiments and more preferred conditions would be selected. Labeled probe DNA fragments used in hybridization can be denatured, for example, under heating conditions at about 70 to 100 °C, preferably about 100 °C, for about 1 to 60 minutes, preferably about 5 minutes, etc. The hybridization is carried out by well known techniques per se in the art or according to methods analogous thereto. As used herein, the stringent conditions refer to, for example, those equivalent to hybridization in about 15 to 50 mM, preferably about 19 to 40 mM, and more preferably about 19 to 20 mM, with regard to Na ion concentration, at about 35 to 85°C, preferably about 50 to 70°C, and more preferably about 60 to 65°C with regard to temperature. After the hybridization is completed, the carriers (such as filters) are washed extensively to remove labeled probes other than the labeled probe DNA fragments which specifically hybridize. Thereafter, detections are done. The filter washing process may be performed by a method suitably selected from techniques used in the art. For example, the washing is carried out in 0.5×SSC solution (× SSC = 0.15M NaCl, 15mM citric acid) containing 0.1% SDS.

The hybridized nucleic acids can be detected representatively by autoradiography, but the detection may be performed by a method suitably selected from techniques used in the art. The nucleic acid bands corresponding to the detected signal are suspended in a suitable buffer solution such as SM solution (50mM Tris-HCl buffer, pH7.5, containing 100mM NaCl and 10mM $MgSO_4$). After the nucleic acid suspension is diluted to a suitable level, target nucleic acids can be isolated and purified. Further, the nucleic acids can be subjected to amplification.

Samples containing the target nucleic acids (e.g., phage particles, recombinant plasmids, recombinant vectors and others) can be isolated and purified by customary techniques used in the art. For instance, they are obtained by glycerol gradient ultracentrifugation (Molecular Cloning, a laboratory manual, ed. T. Maniatis, Cold Spring Harbor Laboratory, 2nd ed. 78, 1989), and other techniques. DNA can be isolated and purified from phage particles and the like by customary techniques used in the art. For instance, the resulting phages are suspended in TM solution (50mM Tris-HCl buffer, pH7.8, containing 10mM $MgSO_4$), etc., and treated with DNase I and RNase A, etc. followed by addition of a mixture solution of 20mM EDTA, 50 μg/ml Proteinase K and 0.5% SDS. The resultant mixture is incubated at about 65°C for 1 hr and subjected to phenol extraction and then to diethyl ether extraction, followed by precipitation with ethanol to form DNA precipitates. Next, the resultant DNA is washed with 70% ethanol, dried and dissolved in TE solution (10mM Tris-HC1 buffer, pH8.0, containing 10mM EDTA). In addition, a large amount of target DNA can be obtained by subcloning, etc. For example, the subcloning can be performed with plasmid vectors, etc. in host E. coli, etc. The DNA thus subcloned can also be isolated and purified by techniques including centrifugation, phenol extraction, ethanol precipitation, etc. in the same manner as aforementioned. The nucleic acids herein are single- and double-stranded DNA, RNA, DNA:RNA hybrids, synthetic DNA, and others. They may be any of genome DNA, genomic DNA libraries, cell-derived cDNA, and synthetic DNA. In accordance with the present invention, through utilizing the findings of a galectin 9 gene structure and a DNA sequence thereof, it is possible to design probes and primers for screening of targeted genome DNA, and mRNA, as well as detection of galectin 9-expressing activity, galectin 9 activity, galectin 9-inducing activity, and the like. Specific detection probes and primers may include those substantially allowing specific detection of galectin 9-inducing activity. Representatives of such species include those allowing the detection of characteristic sequence segments existing in genes as disclosed in WO 02/37114 A1. Preferable species may include those capable of detecting part of said galectin 9 gene as long as they are useful in specific detection. For instance, human galectin 9 DNA can be obtained by PCR with a set of primers,
Gal-9

```
    sense sequence: CAGGCACCCATGGCTCAAACTAC        [SEQ ID NO: 1]


    antisense sequence: TATCAGACTCGGTAACGGGGGT    [SEQ ID NO: 2] ,
```

a set of primers as disclosed in Examples, or other primer pairs. Preferably, probes and primers used in the detection are nucleotide fragments or oligonucleotides, which are required to hybridize specifically with a gene to be targeted. In effective cases for the detection, preferable elements are those which can perform hybrid formation to bind effectively. For such purposes, examples thereof include oligonucleotides containing 5 or more contiguous nucleotide bases, or 10 or more contiguous nucleotide bases; preferably oligonucleotides containing 15 or more contiguous nucleotide bases, or 25 or more contiguous nucleotide bases; and further preferably oligonucleotides (or polynucleotides) containing 30 or more contiguous nucleotide bases, or 50 or more contiguous nucleotide bases. To an oligo-(or poly-)nucleotide containing the nucleotide sequence that can effectively hybridize to a target sequence may be added another nucleotide or nucleotide chain at one end or both ends of said selected nucleotide sequence. It may also be linked with a member selected from labels (including markers and reporters) and others, as disclosed herein. The labels may be those incorporated, for example, in the process of PCR. The label used herein is selected from those widely utilized in the art. Examples of said labels are radioactive substances, fluorescent substances, luminescent substances, enzymes, and the like, as well as biotin-avidin systems, etc. Preferably, the probe may be labeled in order to facilitate detection. In order to isolate genes, PCR, and further PCR coupled with RT (reverse transcriptase) (RT-PCR; reverse transcription-polymerase chain reaction) is applicable. For the purpose of quantitatively assaying, competitive PCR can be conducted. For example, when a predetermined cDNA is used, a particular intracellular gene can be detected or assayed, for instance, by Northern blotting, Southern blotting, in situ hybridization and other techniques. In order to amplify specifically a predetermined gene upon detection, applicable primers are a pair of oligonucleotides defining both ends of a sequence region to be amplified, or a pair of one constituent oligonucleotide of universal primers and another constituent selected from oligonucleotides as disclosed herein, and predetermined specific oligonucleotides as set forth in the present invention.

Said primers are used to initiate the chain elongation of sequences to be amplified. Also, such primers can be applied to not only PCR techniques but also LCR, TAS and other techniques. The application of said primers is not limited to specific nucleic acid amplification, but covers various uses, diverse adaptations, and versatile objects. In said detection, amplification reactions are performed with primers (which allow specific amplification of target genes depending on necessity) obtained according to methods as disclosed in the aforementioned "gene recombination techniques", and whether or not the amplification takes place is monitored. Therefore, known nucleic acid (such as DNA and mRNA) extraction techniques or other suitable nucleic acid extraction techniques can be used in the methods of the present invention. Extracted nucleic acids (such as DNA and mRNA) can be amplified by any amplification technique, including for example PCR, RT-PCR, etc. After amplification operations, the resultant products are subjected to electrophoresis, such as agarose gel electrophoresis, followed by checking the presence or absence of amplified DNA by conventional methods. For example, after staining with an ethidium bromide solution, a stain for DNA can be visualized by UV illumination. Alternatively, a DNA band can be detected with a predetermined probe. For instance, when the galectin 9 gene is not expressed in a sample to be tested, the amplification does not take place or does at a low level and it is therefore possible to use detection methods free of separation of amplified products, such as blotting and reverse blotting.

The related proteins, polypeptides, fragments thereof, and nucleic acids such as DNA (including mRNA and oligonucleotides) as targeted herein can be applicable, alone or in admixture with a variety of the other elements, to the technology of genomics & proteomics, optionally in combination with antisense techniques, antibodies including monoclonal antibodies, transgenic cells (transformants) and other technologies or materials. Thus, the following will be available: gene expression analysis, gene function analysis, protein-protein interaction analysis, and related gene analysis, using nucleic acid arrays and protein arrays. For example, in the nucleic acid array technology, samples are analyzed using cDNA libraries, arranging DNA obtained by PCR on a support plate at a high density with a spotting apparatus, and utilizing hybridization.

The arraying can be performed by immobilization of DNA at each defined site on a support plate such as a slide glass, a silicon plate, and a plastic plate, with needles or pins or using an ink jet printing technique and others. Data are acquired by observation of signals which have resulted from hybridization on the nucleic acid arrays. The signals may be those obtained from labels such as fluorescent dyes (e.g., Cy3, Cy5, BODIPY, FITC, Alexa Fluor dyes (trade name), and Texas red (trade name). Detection can be conducted with a laser scanner and the like. The resultant data may be processed with a computer system installed with programs according to an appropriate algorithm. Also, tagged recombinant expression protein products may be utilized in protein array technology. The instruments and techniques utilizable in the protein array technology may include dimensional electrophoresis (2-DE); mass analysis (MS), including techniques such as electrospray ionization (ESI), and matrix-assisted laser desorption/ionization (MALDI), wherein MALDI-TOF analyzers, ESI-triple quadrupole analyzers, ESI-ion trap analyzers and others may be employed, for protein substances including enzymatically digested fragments; staining techniques; isotope labeling and analysis; image processing techniques; and the like. Therefore, the present invention can encompass softwares, databases and others obtainable or utilizable in the preceding in connection with not only enzyme-gene systems but also antibodies against said targets, and related substances thereof.

In the present invention, detection and measurement (assay) can be carried out by immunostaining including, for example, immunohistochemistry (IHC) staining, immuno-electron microscopy, and immunoassays such as competitive and non-competitive immunoassays. The assays can also be conducted by radioimmunoassay (RIA), FIA, LIA, EIA, ELISA, etc., and with or without B-F separation. The assay is carried out preferably by RIA, EIA, FIA, LIA, and sandwich assays. In an embodiment of the sandwich assay, one of the antibodies is set against this inventive Gal-9 polypeptide or a Gal-9-related peptide fragment while the other directed against a site with the C-terminal residues of galectin-9, wherein one of both the antibodies is detectably labeled (needless to say, other combinations are also possible and may be designed as suitable according to the purpose). The other antibody capable of recognizing the same antigen may be immobilized on a solid phase. As required, incubation is carried out to react sequentially a sample to be assayed, with labeled antibodies, and solid phased antibodies. After the non-binding antibodies are separated, the label is detected or measured. The amount of the measured label is proportional to the amount of an antigen, i.e., the amount of a galectin-9 polypeptide antigen. This assay is referred to as simultaneous sandwich assay, forward sandwich assay, or reverse-sandwich assay, based on the difference according to the addition sequence of the insolubilized antibody and the labeled antibody. For example, washing, stirring, shaking, filtration, pre-extraction for antigen, and other treatments are optionally adopted in the measurement or assay process under specific conditions. The other assay conditions including the concentrations of specific reagents, buffers, and others, temperatures, incubation times, and the like can vary according to elements, such as the concentration of antigens in the sample, or the nature of samples to be measured. Any person ordinary skilled in the art can suitably select and determine optimal conditions effective for each assay while using the general experimentation and perform the selected measurement.

The assay systems for galectin-9 include, for example, protein assay systems, such as systems for immunostaining (METHODS, 24, 289-296(2001); J Immunol Methods, 47(2), 129-144(1981); ibid., 150(1-2), 5-21, 23-32 & 151-158(1992); Cancer J, 7(1), 24-31(2001), etc.) and immunoelectron microscopy (Mol Biotechnol, 7(2), 145-151(1997); J Electron Microsc Tech., 19(1), 57-63 & 64-79(1991); ibid., 19(3), 305-315(1991), etc.), and expression gene assay systems, such as in situ hybridization systems, used effectively for tissues; protein assay systems, such as systems for EIA, RIA, FIA, LIA, and Western blotting (J Electron Microsc (Tokyo), 45(2), 119-127(1996); Methods Biochem Anal., 33, 1-58(1988); Methods Enzymol., 271, 177-203(1996); ibid., 305, 333-345(2000); J Immunol Methods, 152(2), 227-236(1992); ibid., 170(2), 177-184(1994); ibid., 195(1-2), 149-152(1996); Yoshiyuki Kuchino, et al. ed., "Idenshi-Tanpakushitsu, Jikken Sosa Burottingu-ho" (Genes and Proteins, Experimental Procedures, Blotting Methods), Soft Science Co., Ltd., Japan, Nov. 10, 1987, etc.), and expression gene assay systems, such as systems for Northern blotting, dot blotting, RNase protection assay, RT-PCR (reverse transcription polymerase chain reaction), Real-Time PCR (Clinical Chemistry, 46: 11, 1738-1743 (2000)), used effectively for tissue extracts; and protein assay systems, such as systems for EIA, RIA, FIA, LIA, and Western blotting, used effectively for blood and body fluids, etc. Direct assay systems for galectin 9 inducers can also be constructed and used advantageously.

With regard to EIA systems, for example competitive methods utilize solid-phase anti-galectin-9 Ab, a labeled antigen and a non-labeled antigen (the antigen may be galectin-9 or a peptide fragment thereof, etc.), while non-competitive methods, such as sandwich assays, do solid-phase anti-galectin-9 Ab, and labeled anti-galectin-9 Ab, as well as labeled or immobilized antibodies directed to anti-galectin-9 Ab without directly labeling or immobilizing anti-galectin-9 Ab. Sensitivity amplification or enhancement methods include, for example, combinations with non-enzyme-labeled primary Ab, including those using polymers, enzymes, primary Ab (adoptions of Envision reagents; Enhanced Polymer One-step Staining (EPOS)) and combinations with non-enzyme-labeled secondary Ab, including combinations of enzymes with anti-enzyme antibody conjugates such as the PAP (peroxidase-antiperoxidase method), combinations of biotin-labeled secondary Ab with biotin-labeled enzyme-avidin complexes such as the SABC (avidin-biotinylated peroxidase complex method), combinations of biotin-labeled secondary Ab and biotin-labeled enzyme-streptavidin complexes such as the ABC (streptavidin-biotin complex method) and the LSAB (labeled streptavidin-biotin method), combinations of SABC with biotin-labeled tyramide and enzyme-labeled streptavidin such as the CSA (catalyzed signal amplification), methods in which a secondary antibody and an enzyme are labeled with a polymer, etc.

For measurements (and/or detections) according to the present invention, the immunological measurement (immunoassay) is applied. For the measurement (assay), the solid phase carriers used may include various materials and shapes which can be selected from balls, microplates, sticks, microparticles, test tubes, and the like, made of polystyrene, polycarbonate, polypropylene, polyvinyl and other materials, capable of well adsorbing proteins such as antibodies.

The assay can be carried out in a suitable buffer system so as to maintain optimal pH (for example, between pH about 4 and about 9). The particularly preferred buffers may include acetate buffers, citrate buffers, phosphate buffers, Tris buffers, triethanolamine buffers, borate buffers, glycine buffers, carbonate buffers, Tris-HCl buffers, veronal buffers, etc. The buffers can be used optionally in a mixed form at any ratio. Preferably, the antigen-antibody interaction is carried out at a temperature between about 0 and 60°C.

In applying various analytic and quantitative assays including those individual immunological assays (immunoassays) to the measurements (assays) of the present invention, it is unnecessary to set up therefor any special condition, operation, etc. Assay systems for the targets of the present invention or target substances having a substantially equivalent activity thereto may be constructed by adaptations of technical consideration ordinarily given by artisans in the art over general conditions and operations suitable for each of the methods.

For details of those conventional techniques, a variety of reviews, texts, books, etc. may be referred to. They are, for example, Hiroshi Irie (ed.), "Radioimmunoassay", Kodansha Ltd., Japan, 1974; Hiroshi Irie (ed.), "Zoku-Radioimmunoassay" (Radioimmunoassay; Second Edition), Kodansha Ltd., Japan, 1979; Eiji Ishikawa et al. (ed.), "Koso Meneki Sokuteiho" (Enzyme Immunoassays), Igaku-Shoin Ltd., Japan, 1978; Eiji Ishikawa et al. (ed.), "Koso Meneki Sokuteiho" (Enzyme Immunoassays) (2nd Edition), Igaku-Shoin Ltd., Japan, 1982; Eiji Ishikawa et al. (ed.), "Koso Meneki Sokuteiho" (Enzyme Immunoassays) (3rd Edition), Igaku-Shoin Ltd., Japan, 1987; H. V. Vunakis et al. (ed.), "Methods in Enzymology", Vol. 70 (Immunochemical Techniques, Part A), Academic Press, New York (1980); J. J. Langone et al. (ed.), "Methods in Enzymology", Vol. 73 (Immunochemical Techniques, Part B), Academic Press, New York (1981); J. J. Langone et al. (ed.), "Methods in Enzymology", Vol. 74 (Immunochemical Techniques, Part C), Academic Press, New York (1981); J. J. Langone et al. (ed.), "Methods in Enzymology", Vol. 84 (Immunochemical Techniques, Part D: Selected Immunoassays), Academic Press, New York (1982); J. J. Langone et al. (ed.), "Methods in Enzymology", Vol. 92 (Immunochemical Techniques, Part E: Monoclonal Antibodies and General Immunoassay Methods), Academic Press, New York (1983); J. J. Langone et al. (ed.), "Methods in Enzymology", Vol. 121 (Immunochemical Techniques, Part I: Hybridoma Technology and Monoclonal Antibodies), Academic Press, New York (1986); J. J. Langone et al. (ed.), "Methods in Enzymology", Vol. 178 (Antibodies, Antigens, and Molecular Mimicry), Academic Press, New York (1989); M. Wilchek et al. (ed.), "Methods in Enzymology", Vol. 184 (Avidin-Biotin Technology), Academic Press, New York (1990); J. J. Langone et al. (ed.), "Methods in Enzymology", Vol. 203 (Molecular Design and Modeling: Concepts and Applications, Part B: Antibodies and Antigens, Nucleic Acids, Polysaccharides, and Drugs), Academic Press, New York (1991); etc. and references quoted in the above documents, the disclosures of which are incorporated herein by reference.

The Galectin 9-inducer activity is detectable via detecting/measuring the galectin 9-expressing genes (including DNA such as cDNA and RNA such as mRNA) according to the aforementioned "gene recombination techniques", by the known techniques for detecting/measuring the expression of a specific gene in the art, such as in situ hybridization, Northern blotting, dot blotting, RNase protection assay, RT-PCR, Real-Time PCR (Journal of Molecular Endocrinology, 25, 169-193 (2000) and reference documents quoted therein), and DNA array analysis ((Mark Shena (Ed.), "Microarray Biochip Technology", Eaton Publishing (March, 2000)). Galectin 9 expressing gene assay systems, and reagents, methods or processes for their applications, utilizing these techniques, are all encompassed in the present inventive assay reagents and methods for galectin 9-inducer activity, and application systems utilizing the same. The in situ hybridization may include, for example, non-RI in situ hybridization, and may also include, for example, direct and indirect methods. The direct method is based on, for example, direct labels where a detectable molecule (reporter) is directly bound to a nucleic acid probe, whereas the indirect method is based on, for example, indirect ones where a signal is amplified using an antibody against a reporter molecule. Functional groups (e.g., primary aliphatic amino groups, SH groups, etc.) are incorporated into oligonucleotides in the nucleic acid probe, and may be coupled with haptens, fluorescent dyes, enzymes and the like. Representatives of labels for the nucleic acid probes include digoxigenin (DIG), biotin, fluorescein and the like. The labels as used herein can be suitably selected from those described in connection with antibodies as disclosed herein above. Multiple labeling can also be utilized. Further labeled antibodies can also be utilized. Applicable methods of preparing labeled nucleic acid probes are suitably selected from those techniques known in the art, but include, for example, random prime labeling, nick translation, PCR-mediated DNA amplification, labeling/tailing, in vitro transcription, etc. The treated samples can be observed using techniques suitably selected from those known in the art. Examples of such techniques may include dark-field microscopy, phase-contrast microscopy, reflection-contrast microscopy, fluorescent microscopy, digital imaging microscopy, electron microscopy and the like. Furthermore, flow cytometry can be used.

In accordance with the present invention, galectin 9 and galectin 9-expressible genes can be used as markers for galectin 9-inducers, thereby allowing the production of a variety of galectin 9-inducer activity detection agents or reagents for detection and/or assaying of galectin 9-inducers; galectin 9-inducer activity detection methods or methods for detection and/or assaying of galectin 9-inducers; and further galectin 9-inducer activity detection reagent sets or systems, and/or reagent sets/systems for detection and/or assaying of galectin 9-inducers, which are not only useful but also advantageous in purification, identification, isolation and/or utilization of galectin 9-inducers.

The present invention also provides methods for inhibition or suppression of cancer metastasis, reagents or kits for their applications, and applied systems (including detection/assay systems), based on induction of galectin 9 production and release. Antitumor agents, antiallergic agents, immunosuppressants, pharmaceutical agents for auto-immune diseases, anti-inflammatory agents, and active components for adrenal cortical steroid hormone alternatives can be provided by controlling the in vivo levels of galectin 9, and/or the in vivo expression of galectin 9. Also, the galectin 9-inducers can be utilized in the technical field for applications of pharmacological actions and/or biological activities exerted by glucocorticoids. Allergy and autoimmune diseases are raised by immunological overreactions of CD4 positive T lymphocytes, and steroids and immunosuppressants are used to treat therapeutically or prophylactically refractory allergic and auto-immune diseases. Since galectin 9 is apparently involved in these reactions, galectin 9-inducers are expected to exert immunosuppressive, anti-inflammatory and antiallergic actions, thereby allowing applications of antitumor agents, antiallergic agents, immunosuppressants, pharmaceutical agents for auto-immune diseases, anti-inflammatory agents, and adrenal cortical steroid hormone alternatives.

The active components of the present invention [e.g., Gal-9 inducers, liquid solutions containing the same, etc. ] can be employed as pharmaceutical agents usually in the form of a pharmaceutical composition or preparation alone or in admixture with a variety of pharmaceutically acceptable aids. For example, the active components can be administered alone or in the form of a pharmaceutical composition or preparation in admixture with any of various pharmaceutically acceptable aids. Preferably, it may be administered in the form of a convenient pharmaceutical composition or formulation suitable for oral, topical, parenteral application, or the like. Any of dosage forms (including those for inhalation and rectal administration) may be selected depending on purpose. The active components of the present invention can be used in combination with any of various drugs, including antitumor drugs (antineoplastic drugs), tumor metastasis-inhibitors, inhibitors for thrombogenesis, therapeutic drugs for joint destruction, analgesics, anti-inflammatory drugs, and/or immunosuppressants, which can be employed as not being restricted to particular species as long as they serve effectively or advantageously. For instance, they can be optionally selected from those known in the art.

The parenteral administration includes topical, percutaneous, intravenous, intramuscular, subcutaneous, intracutaneous, and intraperitoneal routes. It is also possible to apply the drug directly to affected sites, and, in a certain case, the direct application is suitable. Preferably mammal animals including human can receive the drug orally or parenterally (e.g., intracellularly, intra-tissularly, intravenously, intramuscularly, subcutaneously, intracutaneously, intraperitoneally, intrapleurally, intraspinally, by instillation, enterally, per rectum, by instillation into the ear, eye, or nose by swabbing or application on the teeth, skin or mucosa, etc.). Specific dosage forms of the pharmaceutical preparations and formulations include pharmaceutical solutions, pharmaceutical dispersions, semisolid preparations, particulate preparations, shaped preparations, extractives, etc. Examples of the dosage forms are tablets, coated tablets, sugar coated tablets, pills, troches, hard capsules, soft capsules, microcapsules, implants, powders, pulvis, granules, fine granules, injections, liquids and solutions, elixirs, emulsions, irrigations, syrups, aqueous mixtures, suspensions, liniments, lotions, aerosols, sprays, inhalations, nebula, ointments, plasters, patches, pastes, cataplasms, creams, oleates, suppositories (e.g., rectal suppositories), tinctures, dermatologic waters, ophthalmic solutions, collunariums, auristillae, paints, transfusions, powders for injection solutions, lyophilized preparations, conditioned gels, etc.

The pharmaceutical compositions can be formulated in accordance with conventional techniques. For example, the pharmaceutical composition or formulation may comprise at least one of said compounds (active components including proteins) of the present invention or a salt alone or in admixture with physiologically allowable carriers, pharmaceutically acceptable carriers, adjuvants, vehicles, excipients, diluents, etc. The compound (active component or protein) of the present invention or a salt thereof is usually admixed with a single member selected from the group consisting of physiologically allowable carriers, pharmaceutically acceptable carriers, adjuvants, vehicles, excipients, diluents, flavoring agents, perfuming agents, sweetening agents, expanders, antiseptics, stabilizers, binders, pH regulators, buffering agents, detergents (surfactants), bases, solvents, fillers, bulking agents, solution adjuvants, solubilizers, tonicity agents, emulsifiers, suspending agents, dispersers, viscosity-increasing agents, thickening agents, gelling agents, stiffening agents, absorbents, adhesives, elastomers, plasticizers, disintegrants, aerosol propellants, preservatives, antioxidants, opacifying agents, humectants, emollients, charge protectors, soothing agents, etc., or suitably in a combination thereof, depending on necessity, to give a unit dose form which is required for generally approved pharmaceutical practices.

Formulations suitable for parenteral routes include aseptic solutions or suspensions containing at least one active component in admixture with water or other pharmaceutically acceptable media. Examples of such parenteral formulations are injections. Preferred liquid carriers for injection generally include water, saline, dextrose solution, other related saccharide solutions, ethanol, glycols such as propylene glycol and polyethylene glycol, etc. For the preparation of injections, the active component is usually admixed with any of carriers such as distilled water, Ringer's solution, physiological saline, suitable dispersing agents, moistening agents, suspending agents, and other materials to form injectable formulations including solutions, suspensions, emulsions, etc. by known techniques in the art.

Examples of aqueous liquids for the injection are a physiological saline and isotonic solutions containing glucose and other aids (e.g. D-sorbitol, D-mannitol, sodium chloride, etc.) where they may be used in combination with a suitable pharmaceutically acceptable auxiliary solubilizer such as alcohol (e.g. ethanol, etc.), polyalcohol (e.g. propylene glycol, polyethylene glycol, etc.), nonionic surface-active agent (e.g. Polysorbate 80®, HCO-50, etc.), etc. The injectable oily liquids may include sesame oil, soybean oil, and the like, where they may be used in combination with benzyl benzoate, benzyl alcohol, and other materials as auxiliary solubilizers. In addition, buffers (e.g. phosphate buffer, sodium acetate buffer, etc.) or agents for osmoregulation, analgesic agents (e.g. benzalkonium chloride, procaine hydrochloride, etc.), stabilizers (e.g. human serum albumin, polyethylene glycol, etc.), preservatives (e.g. benzyl alcohol, phenol, etc.), antioxidants such as ascorbic acid, absorbefacients, etc. may be admixed therewith too. The prepared injection solution is usually filled in suitable ampoules.

For parenteral administration, solution or suspension unit dosage forms are prepared in pharmaceutically acceptable sterile fluids such as water, ethanol, and oils, in admixture with or without detergent and other pharmaceutically acceptable aids. The oily vehicle and solvent used in the parenteral formulation may include natural, synthetic or semi-synthetic mono-, di-, or triglycerides; natural, semi-synthetic or synthetic fats and oils; and fatty acids. Examples of such oily vehicles and solvents are plant oils such as peanut oil, corn oil, soybean oil, and sesame oil. For example, this injection can usually be prepared to form unit doses each containing approximately from 0.1 to 10 parts of the compound of the present invention per 100 parts by weight of the dose composition.

The formulation suitable for topical use, such as buccal or rectal application, includes mouthwashes and gargles, dentifrices, sprays for buccal cavity, inhalants, ointments (salves), dental fillers, dental coating agents, dental pastes, suppositories, etc. The mouthwashes and other dental agents are prepared by conventional techniques, using pharmaceutically acceptable carriers. For the sprays for buccal cavity and inhalants, the compound of the present invention can be applied to teeth or other sites after dissolving alone or together with pharmaceutically acceptable inert carriers, in an aerosol or solution for nebulizers, or in the form of powders for inhalation. The ointments (salves) are prepared by conventional techniques, in admixture with conventionally employed pharmaceutical bases such as ointment bases (white petrolatum, paraffin, olive oil, macrogol 400, macrogol ointment, etc.).

The pharmaceutical drugs for topical application (including painting) to teeth and skin can be prepared in the form of a solution or suspension utilizing suitably sterilized water or non-aqueous vehicles. The additives used include buffering agents such as sodium bisulfite and disodium edetate; preservatives including antiseptic, antimicrobial and antifungal agents such as acetic acid, phenylmercuric nitrate, benzalkonium chloride and chlorhexidine; and thickeners such as hypromellose.

The suppositories can be prepared by conventional techniques utilizing carriers well known in the art, preferably suitable non-irritative excipients. Examples of the excipients are those which are solid at room temperature but liquid at rectal temperature wherein such substances melt in the rectum to deliver a drug, such as polyethylene glycols, lanolin, cacao butter, and fatty acid triglycerides. In the suppositories, the compounds of the present invention are applied in the form of compositions containing the same at approximately 0.1 to 95 wt%. The compound, depending on the vehicle and concentration used, can be either suspended or dissolved in the vehicle. Adjuvants such as a local anesthetic, preservative and buffering agent can be dissolved in the vehicle. The formulations suitable for oral application include solid compositions such as tablets, pills, capsules, powders, granules, and troches; fluid compositions such as solutions, syrups, and suspensions; etc. In preparing oral formulations, pharmaceutical adjuvants known in the art are employed. The tablets and pills can be prepared further by enteric coating. When the unit dosage form is a capsule, fluid carriers such as fats and oils can be contained in addition to the aforementioned materials.

When the active components are proteins or polypeptides, conjugation to polyethylene glycol (PEG) is particularly useful, because its toxicity is extremely low in mammals. Further, the conjugation with PEG can sometimes reduce the immunogenicity and antigenicity of a heterologous compound effectively. The compound may be given after being put in a microcapsule device. A polymer such as PEG can be easily attached to an $\alpha$-amino group of amino-terminal amino acids, an $\varepsilon$-amino group of lysine side chains, a carboxyl group of aspartic acid or glutamic acid side chains, an $\alpha$-carboxyl group of carboxyl-terminal amino acids, or an activated derivative of glycosyl chains attached to certain asparagine, serine or threonine residues. Various activated forms of PEG suitable for direct reaction with proteins are known. PEG reagents useful for reaction with amino groups of a protein include active esters of carboxylic acids and carbonate derivatives, particularly those having N-hydroxysuccinimide, p-nitrophenol, imidazole, or 1-hydroxy-2-nitrobenzene-4-sufonate as a leaving group. Similarly, PEG reagents having an aminohydrazine or hydrazide group are useful for reaction with aldehydes produced by periodate oxidation of proteins.

Dose levels of said active components may vary within a wide range. Specific dose levels and administration cycles for any particular patient will be employed depending upon a variety of factors including the activity of specific compounds employed, the sex, age, body weight, general health, diet, time of administration, route of administration, rate of excretion, drug combination, and the severity of the particular disease undergoing therapy.

For the manufacture of pharmaceutical compositions and preparations, the additives, other materials, preparation methods and the like can be suitably selected from those disclosed in Nippon Yakkyokuho Kaisetsusho Henshu Iinkai (Ed.), "14th Edition Nippon Yakkyokuho Kaisetsusho (Commentary on The Japanese Pharmacopoeia 14th Edition (JPXIV))", June 27, 2001, Hirokawa Pub. Co., Tokyo, Japan; Hisashi Ichibagade et al. (Ed.), "Iyakuhin no Kaihatsu (Pharmaceutical Research and Development, Ikuo Suzuki, chief editor), Volume 12 (Seizai Sozai I (Pharmaceutical Necessities 1))", October 15, 1990, Hirokawa Pub. Co., Tokyo, Japan; ibid., Volume 12 (Seizai Sozai II (Pharmaceutical Necessities 2)), October 28, 1990, Hirokawa Pub. Co., Tokyo, Japan; etc., depending on necessity, and can be adapted by referring to the disclosures therein.

The active components of the present invention are useful and advantageous in view of, as disclosed herein, controlling biological activities carried by galectin 9 through induction of galectin 9 production and/or release to utilize galectin 9 properties, i.e., for example,

human galectin 9 is non-cytotoxic against normal cells but cytotoxic against tumor cells,

human galectin 9 induces apoptosis in tumor cells but does not in normal cells,

human galectin 9 inhibits or suppresses malignant cell metastasis, and/or

human galectin 9 induces apoptosis in activated immune cells, in particular, activated CD4 positive T cells while it does not in resting T cells, notably in resting CD4 positive T cells (helper T cells).

Said active components are promising to act as antitumor agents (antineoplastic agents), antiallergic agents, immunosuppressants, pharmaceutical agents for auto-immune diseases, anti-inflammatory agents, and drugs utilizing the same activity as that owned by adrenal cortical steroid hormones.

Cytotoxicity induced by natural killer cells can be assayed herein. Applicable assay methods for natural killer (NK) cell cytotoxicity induced by stimulation with active substances are selected from those known in the art and can be performed using any commercially available kit. The commercially available kit includes, for example, LDH Cytotoxicity Detection Kit (TaKaRa, Japan), etc. A representative of said cytotoxicity assay is a simple colorimetric assay method to quantitate cytotoxicity/cytolysis based on the measurement of lactate dehydrogenase (LDH) released from damaged cells into cell culture supernatants. LDH does not pass through cell membranes under normal conditions, but is released outside of cells, i.e., into the culture medium, upon damage of the cytoplasmic membrane. The activity of released LDH is determined in an enzymatic test based on the LDH-catalyzed dehydrogenation of lactate to form pyruvate and NADH. The resultant NADH is used for diaphorase-catalyzed reduction of tetrazolium salts which are converted into red formazan dyes showing the absorption at 490 nm. The enzyme activity can be measured according to the increase of measured absorbance values at 490 nm. In this technique, an increase in the number of dead or plasma membrane-damaged cells leads to an increase of the LDH enzyme activity in the culture supernatant. This allows the measurement of cytotoxicity.

In another assay, mononuclear leukocytes are obtained, an aliquot ($3 \times 10^6$ cells/mL) of the mononuclear leukocytes is stimulated with an active substance (e.g., BALL-mf, IL-2, etc.), or non-stimulated (control; e.g., treated with PBS), and cultured in an appropriate medium (e.g., 10% FCS containing RPMI 1640 supplemented with an antibiotic antimycotic solution (Sigma chemicals, St. Louis, MO, USA). After cultivation, the resulting cells are used as effector cells for target cells.

Target cells K562 cells are treated with $Na_2{}^{51}CrO_4$ (Daiichi Radioisotope Laboratories, Tokyo, Japan; specific activity, 1 mCi/mL) to give labeled cells (50 $\mu$Ci/$10^6$ cells). The resultant cells are washed twice, and then incubated at 37°C for 30 min. After washing, the cells are resuspended to adjust the cell suspension to a concentration of $1 \times 10^5$ cells/mL. The labeled cells are transferred into each well of a 96-well round bottom microtiter plate ($1 \times 10^4$ cells/well, 3 sets) and then incubated together with effector cells at a level of effector cell : target cell ratio (E:T ratio), 10 to 40. Target cells incubated in medium alone are used to monitor the spontaneous release of Cr, and target cells incubated in 1N HCl-added medium are used to monitor the maximum release of Cr. The plate is incubated at 37°C for 4 hr, and subjected to centrifugation at 350xg for 6 min to give a supernatant (100 $\mu$L). The resultant supernatants are assayed with a gamma counter (Aloka, Tokyo, Japan) for their radioactivity.

Calculation of percentage cytolysis:

$$\text{Cytolysis (\%)} = \frac{\text{Exp. Value} - \text{Low Control}}{\text{High Control} - \text{Low Control}} \times 100$$

Remarks:

Exp. Value: experimental release
Low Control: spontaneous release
High Control: maximum release

Cytotoxic data values are expressed as means $\pm$ SE of measurements. The statistical level of significance of the difference between sample means can be evaluated with the use of the Student's t-test.

For terms (words), symbols and/or abbreviations used in the specification and in the drawings, they must conform to the "IUPAC-IUB Commission on Biochemical Nomenclature" or are based on the meanings of the definitions or standards which are commonly or conventionally used in the art.

Examples

Details of the present invention are described by the following examples but such examples are provided only for illustrative purposes, and for referential embodiments of the present invention. These examples have been described herein for the purpose of illustrating specific embodiments of the present invention but should in no way be construed as limiting and restricting the scope of the invention disclosed herein. It should be understood in the present invention that various embodiments can be made or executed within the spirit, scope and concept disclosed herein. All the examples were carried out or can be carried out, unless otherwise disclosed herein specifically, by standard techniques which are well known and conventional to those skilled in the art.

Specific molecular biological operations, treatment conditions, etc. in examples as described herein below are conducted or selected according to customary techniques disclosed in standard experimental manuals, J. Sambrook, E. F. Fritsch & T. Maniatis, "Molecular Cloning", 2nd ed., Cold Spring Harbor Laboratory, Cold Spring Harbor, N. Y. (1989) and D. M. Glover et al. ed., "DNA Cloning", 2nd ed., Vol. 1 to 4, (The Practical Approach Series), IRL Press, Oxford University Press (1995) for DNA cloning; and H. A. Erlich ed., PCR Technology, Stockton Press, 1989 ; D. M. Glover et al. ed., "DNA Cloning", 2nd ed., Vol. 1, (The Practical Approach Series), IRL Press, Oxford University Press (1995) and M. A. Innis et al. ed., "PCR Protocols", Academic Press, New York (1990) for PCR, and others. When commercially available reagents and kits are used, protocols, drugs, etc. attached thereto are employed herein.

Example 1

[Solubilization of Tumor Cell Membranes]

Tumor cells were used to prepare their soluble cell membrane fractions. The tumor cells were human lymphoid B cell lines, BALL-1 cells, and Daudi cells, which are B lymphoma cells. Solubilizations were performed according to modifications of methods disclosed in Hirashima, M. et al., Immunol. Letters, 36: 273-281 (1993) and Seki, M. et al., Int. Arch. Allergy Immunol., 114: 2-5 (1997). BALL-1 cells cultured in 10% FCS-containing RPMI1640 medium were used as starting sources. Harvested BALL-1 cells were resuspended in 1 mM phenylmethylsulfonyl fluoride (PMSF)-PBS ($1 \times 10^9$ cell/5 mL). Four freeze-thawing cycles were carried out with liquid nitrogen and water at room temperature (freeze-thawingx4 cycles). Next, sonication (output=4, duty cycle%=50, on ice, for 4 min (substantial period: about 2 min)) was conducted. The sonication was operated in such a manner that samples were sonicated for 2 min, next rested, and then resonicated for 2 min. The disrupted products were centrifuged. The centrifugation was performed at 100,000 G for 1 hr at 4°C.

Pellets from the centrifugation step were refloated in a solution consisting of 50 mM Tris-HCl (pH 8.2), 1 mM EDTA and 1% CHAPS at a volume equivalent to the volume at which the BALL-1 cells were resuspended in 1 mM PMSF-PBS as aforementioned, and homogenized. The homogenization was conducted with a 10 mL or 20 mL Teflon@ glass homogenizer on ice for several minutes until pellets disappeared completely. The resultant products were centrifuged. The centrifugation was performed at 20,000 G (15,000 rpm) for 30 min at 4°C. Supernatants were collected (Sup(MF)). The optical density (OD) was measured. A blank run used is a solution consisting of 50 mM Tris-HCl (pH 8.2), 1 mM EDTA, and 1% CHAPS. The resultant supernatant was dialyzed thoroughly against PBS, and then passed through a porous filter (pore size: 0.2 $\mu$mL) to afford a soluble tumor cell membrane fraction (mf, i.e., BALL-1 mf). The mf was stored at -80°C until use.

Similarly, Daudi cells cultured in 20% FCS-containing RPMI1640 medium were treated to give mf (Daudi mf), which was stored at -80°C until use.

[Preparation of Tumor Cells]

Target tumor cells used were sarcoma Meth-A cells. Sarcoma Meth-A cells were maintained in RPMI 1640 medium containing 10% fetal bovine serum (FBS), 100U/mL penicillin, 100 $\mu$g/mL streptomycin, and 0.25 $\mu$g/mL amphotericin B. Cultured Meth-A cells (1x10$^{16}$ cells/100 $\mu$L PBS) were inoculated subcutaneously into the back of Balb/c mice. Three weeks later, the grown tumors were excised, and cut into 2 cm size pieces, which were then placed in 10% FBS-containing RPMI 1640 medium supplemented with 1 mg/mL collagenase (type I, Sigma C-0130; Sigma, St. Louis, MO, USA). While constantly stirring with a magnetic stirrer, the mixture was homogenized at 37°C for 1.5 hr, passed through a layer of cotton gauze, then washed twice with PBS, and next resuspended in PBS (2x10$^6$ cells/mL; viable cell percentage, about 90%).

[Tumor Growth and Rejection]

Meth-A cells (1x10$^5$ cells/50$\mu$L) were inoculated subcutaneously into BALB/c mice (7-week-old male, n=10/group) at the dorsal surface. Immediately after the inoculation, 100 ng/200$\mu$L of BALL-mf or Daudi-mf was injected subcutaneously into the mice at the periphery of said tumor cell inoculated site (100 $\mu$L/site). PBS was used as a control group. The mf or PBS was injected every 3 days. The body weight of said animals and the size (short axis, a and long axis, b) of said tumors were measured at a cycle of 3 times per week. Each tumor volume was calculated by the following equation:

$$V \ (mm^3) \ = \ 0.4 \ \times \ a \ \times \ b^2$$

according to the method disclosed in Attia et al., Cancer Res., 26: 1787-1800 (1966).

[RT-PCR for Galectin 9]

Total RNA was isolated with TRIzol® Reagent (Gibco, BRL) from BALL-mf, Daudi-mf or PBS treated cells. One-step reverse transcription reaction was performed using Gene Amp RNA PCR Kit (Perkin Elmer) with 0.5 $\mu$g of total RNA to give DNA products. Next, a polymerase chain reaction (PCR) was performed to amplify mouse galectin 9, human galectin 9, and GAPDH transcripts, respectively. The reverse transcription (RT) and PCR were conducted according to the protocol supplied with the kit. In brief, primers used for the reactions are the following:

Human galectin 9
Sense sequence, hG9S:

CGTCAATGGCTCTGTGCAGCTGTC 〔SEQ ID NO: 3〕

Antisense sequence, hG9AS:

AGATCCACACTGAGAAGCTCTGGC 〔SEQ ID NO: 4〕

Mouse galectin 9
Sense sequence, mG9SQ1:

GGTCAGAGTTCAAGGTGATGGTGA 〔SEQ ID NO: 5〕

Antisense sequence, mG9SQ2:

GCCTGATATCATGATGGACTTGGA 〔SEQ ID NO: 6〕

which were synthesized through Amersham Pharmacia Biotech. A PCR cycle was repeated 30 times to obtain amplified transcripts. All the reactions were conducted in GeneAmp® PCR System 9600 (Perkin Elmer Applied Biosystems).

PCR products were applied to 1.5% agarose gel containing ethidium bromide (1$\mu$g/mL) for visualization with UV. The respective PCR products were purified. Sequencing of galectin 9 PCR products was carried out with ABI PRISM® BigDye® Terminator Cycle Sequencing Ready Reaction Kit (Perkin Elmer Applied Biosystems). In each reaction, the following reagents were added to a tube: 8 $\mu$L Terminator Reaction Mix, 500 ng PCR product, 3.2 pmol primer for Gal-9, and deionized water. DNA sequencing was carried out on GeneAmp® PCR System 2400. Each sequencing result from both of low and high molecular band PCR products was corresponding to one of galectin 9 sequences different in linker peptide domain length. Finally, the intensity of bands was measured with NIH image 1.61 program.

[Western Blotting]

(1) Preparation of Purified Recombinant Human Galectin 9CT-Specific Antibody from Rabbit Anti-Recombinant Human Galectin 9CT Serum

A purified polyclonal antibody (polyclonal antibody against human Gal-9; polyclonal anti-Gal-9 Ab) was obtained from a rabbit immunized with the C-terminal domain of human Gal-9 (Gal-9CT). The resultant antibody (Ab) was prepared through purification on Gal-9 C-terminal domain-coupled Sepharose®. It has been verified that said Ab recognizes mouse Gal-9.

1. Ammonium Sulfate Fractionation of Antiserum (preparation of crude IgG Fractions)

To a glass beaker was added antiserum (rabbit anti-recombinant human Gal-9CT serum, 100 mL) and phosphate-buffered physiological saline (hereinafter, abbreviated to PBS, 100 mL) under ice cooling, and an aqueous saturated ammonium sulfate solution (100 mL) was then added dropwise at a rate of 5 mL/min while the mixture was stirred with a 30 mm Teflon® stir bar on a magnetic stirrer. After completion of aqueous saturated ammonium sulfate solution addition, stirring was continued for an additional 30 min. The resultant solution was transferred into a centrifugation tube, and then centrifuged at 13,000 rpm (RPR-16 rotor, 17,000 x G, high-speed centrifuge, Hitachi Koki Co., Ltd., Japan) for 30 min (4°C; hereinafter centrifuged at 4°C unless otherwise disclosed herein specifically). After the supernatants were discarded, precipitates were dissolved by addition of 100 mL PBS (ice-cooled; hereinafter ice-cooled PBS was used unless otherwise disclosed herein specifically). The resulting solution was transferred into a beaker containing a 20 mm Teflon® stir bar. An aqueous saturated ammonium sulfate solution (67 mL) was added dropwise under ice cooling in the same manner as aforementioned, and stirring was done for an additional 30 min. The resultant solution was transferred into a centrifugation tube, and then centrifuged at 13,000 rpm (RPR-16 rotor, high-speed centrifuge, Hitachi Koki Co., Ltd., Japan) for 30 min. After the supernatants were discarded, precipitates were dissolved by addition of PBS (50 mL). The resultant solution was placed in a dialysis tube (Dialysis Membrane 27, Wako Pure Chemical Industries, Ltd., Japan), and dialyzed against PBS. After dialysis, the dialysis tube solution was transferred into a centrifugation tube, and centrifuged at 13,000 rpm (RPR-16 rotor) for 30 min. To the resultant supernatant was added 10% (w/v) sodium azide at a ratio of 0.05 mL/10 mL (sodium azide/supernatant), and the mixture was stored at 4°C in a plastic bottle (crude IgG fraction).

2. Affinity Purification on Antigen Column

To the crude IgG fraction (50 mL) prepared in the above step 1 was added PBS(containing 40 mmol/L lactose and 0.05% (w/v) sodium azide) at a ratio of 1:1 (IgG fraction : PBS) to form a diluted crude IgG fraction solution. A GST-recombinant galectin-9CT (GST-rGal-9CT) (10 to 20 mg)-coupled HiTrap® NHS-activated column (5 mL, Amersham Biosciences) was connected to a peristaltic pump, and equilibrated by washing the column with 20 mL of PBS (containing 20 mmol/L lactose) (flow rate: 2 mL/min). The diluted crude IgG fraction solution was loaded on the equilibrated column (flow rate: 1 mL/min), and the first 5 mL flow-through fraction was discarded. Next, the subsequent flow-through fractions were collected in a plastic bottle. After the crude IgG fraction was passed through the column, 5 mL of PBS was next passed through the column, and the eluate was collected in the same plastic bottle. The above collected plastic bottle solution was passed through the column under the same conditions, and the flow-through fractions were collected in a plastic bottle. Next, the column was washed with 50 mL of PBS (containing 20 mmol/L lactose) (flow rate: 2 mL/min). The final 2 mL flow-through fraction was collected in a test tube and assayed for absorbance at 280 nm, relative to a control, PBS (containing 20 mmol/L lactose). When the absorbance was over 0.02, the column was washed with additional 10 mL PBS (containing 20 mmol/L lactose). This step was repeated until the absorbance returns to 0.02 or below when the absorbance of the final 2 mL flow-through fraction was measured.

Next, 30 mL of 0.2 mol/L glycine-HCl (pH 2.5) was passed through the column (flow rate: 1 mL/min), and eluates were collected in 2-mL fractions. Each fraction was assayed for absorbance at 28 nm and fractions with the absorbance of 0.1 or above were joined to form one sample fraction. The pH of this eluate fraction was adjusted to 7 to 7.5 with 1mol/L 2-amino-2-hydroxymethyl 1,3-propanediol (hereinafter, referred to as Tris) in combination with a pH meter. The column was equilibrated with 40 mL of PBS (containing 0.05% (w/v) sodium azide, flow rate: 2 mL/min), and stored at 4°C. The eluate fraction was placed in a dialysis tube (Dialysis Membrane 20, Wako Pure Chemical Industries, Ltd., Japan), and dialyzed against PBS (4°C). The dialysis tube liquid was transferred into a centrifugation tube, and centrifuged at 13,000 rpm (RPR-18 rotor, 17,000 x G, high-speed centrifuge, Hitachi Koki Co., Ltd., Japan) for 30 min. To the resultant supernatant was added 10% (w/v) sodium azide at a ratio of 10 mL : 0.1 mL (supernatant : 10% (w/v) sodium azide), and stored at 4°C in a plastic bottle (affinity-purified anti-Gal-9CT Ab).

3. Removal of rGal-7 Cross-Reactive Ab from Affinity-Purified Anti-rGal-9CT Ab

A GST-recombinant galectin-7 (GST-rGal-7, 5 to 10 mg)-coupled HiTrap® NHS-activated column (5 mL, Amersham Biosciences) was connected to a peristaltic pump, and washed with 20 mL of PBS (flow rate: 2 mL/min). The affinity-purified anti-rGal-9CT Ab from the above step 2 was loaded on the GST-rGal-7-coupled column (flow rate: 0.5 mL/min), and the first 4 mL flow-through fraction was discarded. Next, the subsequent flow-through fractions were collected in a plastic bottle. After the affinity-purified anti-rGal-9CT Ab was passed through the column, 5 mL of PBS was next passed through the column, and the eluate was collected in the same plastic bottle. The above collected plastic bottle flow through solution was passed through the column under the same conditions, and the flow-through fractions were collected similarly in a plastic bottle. The 280 nm absorbance was measured and the product stored at 4°C (end sample product: purified rGal-9CT Ab). Bound Gal-7 cross-reactive Ab is obtained by passing 0.2 mol/L glycine-HCl (pH 2.5) through the column (flow rate: 1 mL/min) .

(2) Immunostaining

To cell pellets was added a lysis buffer (10 mM Tris-HCl, 0.15 M NaCl, 2 mM EDTA, 2 mM EGTA, freshly-added 0.5 mM PMSF, 10μg/mL leupeptin, antipain, pepstatin A, and 1 mM DTT), and the mixture was sonicated to give a cell lysate. To the cell lysate was added SDS, the sample mixture heated at 100°C for 5 min, and then placed on ice. Each sample was applied on 12% acrylamide-SDS gels, and separated proteins were transferred onto a PVDF membrane (BioRad Laboratories). A 5% skim milk solution in 0.1% Tween-20-containing PBS (PBS-T) was used to block nonspecific binding. Said PVDF membrane was washed with PBS-T several times, and then incubated with a dilution of 10$\mu$ g/mL purified anti-rGal-9CT Ab in PBS-T for 1 hr. After washing, said PVDF membrane was incubated with PBS-T containing peroxidase-coupled goat anti-rabbit IgG (Amersham Pharmacia Biotech) for 45 min. Said PVDF membrane was dipped in ECL®-HRP substrate solution contained in ECL® Kit (Amersham Pharmacia Biotech), and immunoblot bands were visualized by exposing the membrane to an XJB-1 X-ray film (Kodak).

[Flowcytometry]

In order to examine the expression of cell surface-bound galectin-9, cells were collected by centrifugation, washed with PBS containing 0.05% NaN$_3$ and 2% fetal calf serum (FCS) (PBS+), and incubated in the presence of 25 $\mu$ g/mL rabbit anti-hGal-9 Ab on ice for 30 min. After the cells were washed several times with PBS+, the cells were incubated in the presence of FITC-coupled goat anti-rabbit IgG Ab (Santa Cruz Biotechnology) on ice for 30 min. The examination of cytoplasmic Gal-9 expression was done by slight modifications of the methods disclosed in Jacob, M.C. et al., Cytometry, 12: 550-558 (1991), and Sumner, H. et al., J. Immunol. Methods, 136: 259-267 (1991). In brief, the cells were fixed with ice-cooled PBS containing 4% paraldehyde for 10 min. After washing with PBS+, the cells were admixed with 25 $\mu$g/mL rabbit anti-hGal-9 Ab in saponin buffer (PBS, pH 7.4 containing 0.1% saponin and 0.01 M HEPES buffer), next incubated at room temperature for 30 min, and then incubated together with FITC-coupled goat anti-rabbit IgG Ab (Santa Cruz Biotechnology) on ice for 30 min. All of cells gated according to the setting of scatter gauge (15000 events) were analyzed with COULTER® EPICS XL-MCL$^{TM}$ Flow Cytometer using SYSTEM II® Software Version 1.0 for Gal-9 staining. A suspension of Flow-check® fluorospheres (COULTER Corporation) was used to verify the optimal alignment of the optical and fluidic systems of the flow cytometer.

[Histopathological Analysis]

On day 27 post-inoculation of tumor cells, the tumor was excised and its weight was measured. After histopathological examination samples were fixed with 10% neutral buffered formaldehyde solution, then paraffin embedded tissue was cut into sections 4 $\mu$ m thickness, deparaffinized, hydrated, and stained with hematoxilin-eosin or Giemsa stain (Giemsa's reagent).

[In Stu Hybridization]

In situ hybridization was performed to examine whether or not galectin 9 mRNA was contained in cells accumulated at BALL-mf injected sites. Digoxigenin (DIG)-labeled RNA probes were synthesized by in vitro transcription with DIG RNA Labeling Kit (SP6/T7; Roche Molecular Biochemicals, Mannheim, Germany). PCR-amplified Gal-9 cDNA fragments (nucleotides at positions 500 to 1208 of the Gal-9 nucleotide sequence; Matsumoto, R. et al., J. Biol. Chem., 273: 16976-13984 (1998)) were cloned into pGEM-T Easy Vector (Promega, Madison, WI, USA). Linearized plasmid DNA was used as a template DNA for in vitro transcription. Sense and antisense probes were synthesized. The sense probe was used as a negative control. Hybridization protocols were applied to 4 $\mu$m paraffin sections and the operation was done according to the kit manufacturer's protocol. The section was digested with proteinase K at 37°C for 2 hours, then hybridized with probes (1$\mu$g/mL) in 20$\mu$L of hybridization buffer at 43°C overnight under a cover slip. Digoxigenin (DIG) labels were visualized with DIG DNA Labeling and Detection Kit (Roche Molecular Biochemicals). The control groups are those using said sense probes and those where probes were eliminated.

[Results]

[Tumor Growth Curve and Tumor Rejection Percentage]

After Balb/c mice were inoculated with Meth-A sarcoma cells, the efficacy of BALL-mf, Daudi-mf, and PBS on the tumor growth in the animals was examined, respectively. As a result, the tumor cells were grown for initial 2 weeks equally in all the inoculated animal groups (Fig. 1(a)). In both the Daudi-mf treated group and PBS treated group, the tumor cells still continued growing afterward. There was no significant difference in tumor size between these two mouse groups (Fig. 1(a)). In contrast, for BALL-mf treated mice, the tumor size began to turn into reduction two weeks later. Eighteen days later, the tumor size became significantly smaller than in the Daudi-mf and PBS treated mouse groups (Fig. 1 (a)). Meanwhile there was no noticeable difference in body weight among these 3 mouse groups during experimentation. The tumor was first observed to be rejected in one mouse among 10 animals on day 20 after treatment with BALL-mf, subsequently in 3 additional mice on day 22, and afterward in 4 additional mice on day 25. The tumor was completely rejected in 8 BALL-mf treated mice among 10 animals on day 27 while it was barely in one animal among PBS and Daudi-mf treated mouse groups.

These results indicate apparently that BALL-mf has antitumor activity (Fig. 1 (b), chi-square ($\chi^2$) analysis: p=0.0006).

[Histopathological Examination]

Tissue samples were histopathologically examined, and cell responses were disclosed in the tumor periphery at the BALL-mf injected site. As shown in Fig. 2a, the formation of granulation tissue composed of predominant eosinophils (arrows with E) and mononuclear cells, accompanied with few neutrophils, was observed at the injected site in BALL-mf injected mice. The granulation tissue was observed even in the Daudi-mf treated mice; however, most of infiltrated cells were mononuclear cells but they were not eosinophils (Fig. 2b). A large number of mast cells were found at connective tissues over or underneath the cutaneous muscle layer of the BALL-mf injected site while only few mast cells were present at connective tissues over the cutaneous muscle layer of the Daudi-mf injected site.

Further, tissue samples at the periphery of tumors were histopathologically examined. As shown in Fig. 3a, regions with infiltrated inflammatory cells (predominant eosinophils [arrow with E] and a few mast cells [arrow with M] but not neutrophils) were found in the tumor periphery or tumor tissue of BALL-mf treated mice (Fig. 3a). Tumor cells with pyknosis were also found (with an arrow alone, Fig. 3a). As shown in Fig. 3b, the accumulation of metachromatic mast cells were ascertained in the tumor periphery or tumor tissue.

As compared, cellular infiltration was more marked in the tumor periphery of Daudi-mf treated mouse tissue (Fig. 3c); surprisingly, however, countless neutrophils (arrow with N) and mononuclear cell were observed in the tumor periphery of tissue. Few eosinophils and mast cells were detected at said site. No pyknotic tumor cells were observed (Fig. 3c).

[In Situ Hybridization]

In order to determine what kind of cells were expressing galectin 9 at the injected site, in situ hybridization was conducted. As a result, the infiltration of eosinophils was found underneath the panniculus carnosus muscle of subcutaneous tissue. It was found that at said site galectin 9 was produced mainly in mast cells, and other cells including fibroblasts, lymphocytes, and eosinophils (Fig. 6a). Although mast cells do not usually appear at the periphery of normal panniculus carnosus muscles, the infiltration of Gal-9-containing mast cells was observed at the panniculus carnosus muscle site when injected with BALL-mf (Fig. 6b). As shown in Fig. 6a, Gal-9 mRNA-expressing cells were found at the BALL-mf injected site. It appears that said intensely Gal-9 mRNA-expressing cells are mast cells, according to morphologic or Giemsa staining examinations (Figs. 6a & 6b). Gal-9 mRNA was also expressed in mononuclear cells, eosinophils, fibroblasts, and others at said site; however, it was at by far lower levels than in mast cells (Fig. 6a). In contrast, Gal-9 positive cells were scarcely observed at Daudi-mf injected sites (Fig. 6c).

When Meth-A sarcoma-bearing mice were in vivo treated with BALL-mf, the eradication of tumors was observed. Said tumor eradication is probably attributable to the activation of natural killer (NK) cells and the enhancement of Gal-9 production and/or release. Additionally, eosinophils increased in the tumor periphery. It is well-known that there is a relationship between the prognosis of malignant tumors and the type of cells infiltrated into the supporting tissue of tumors. For instance, a good course and/or outcome of the disease will take place in patients with lymphocytes infiltrated into the periphery of tumors. It may be possible that this results from the production of lymphokines and/or the activation of NK cells.

Although it may be probable that the good course and/or outcome of the disease is associated with the eosinophil increase in the supporting tissue of tumors, it seems that the ill course and/or outcome of the disease is correlated with a high neutrophil : lymphocyte ratio in tissue with increased neutrophils and/or in peripheral blood. One of these explanations is that eosinophils are more cytotoxic than neutrophils, and it may be probably attributed to eosinophil peroxidase-dependent hydroxy radical production. It has also been disclosed that the adhesion of tumorcidal eosinophils to tumor cells is associated with the activation of protein kinases.

Eosinophils were observed to infiltrate into the tumor periphery of BALL-mf treated mice but no neutrophils. In contrast, the infiltration of neutrophils was mainly induced in Daudi-mf treated mice (Fig. 2a & Fig. 2b).

It is thought that the eosinophil increase in the BALL-mf induced tissue is correlated with induced Gal-9 at said site. Up to now, the present inventor and colleagues have found that Gal-9 is one of galectin family members and acts as a novel and significantly potent eosinophil chemoattractant.

Besides eosinophils, the infiltration of mast cells was induced in the tumor and the periphery of BALL-mf injected sites (Figs. 2 & 6). It has already been suggested that mast cells are perhaps related to the good course and/or outcome of the disease, similarly to eosinophils. Additionally, it has already been suggested that eosinophils are possibly involved in IL-4 mediated antitumor activity. Up to now, the present inventor and colleagues have disclosed that short-term stimulation with IL-4 results in an increase in the PPD-induced production of Gal-9, but a decrease in the production of IL-5 from peripheral mononuclear cells. Since mast cells are main potential sources for IL-4 at inflammatory sites, it may be possible that mast cells at said sites are involved in the accumulation of eosinophils. When based on in situ hybridization results, it seems that mast cells are important sources for Gal-9 at BALL-mf treated sites (Fig. 6).

Up to now, the present inventor and colleagues have disclosed that co-incubation of human peripheral mononuclear cells with radiated BALL-1 cells results in an increase in NK activity [against not only tumor cell line K562 (NK-sensitive cell), but also other tumor cell lines (e.g., LAK-sensitive cell, Daudi, KMG-2 (glioblastoma cell line), KATOIII (stomach cancer), etc.) ] . In accordance with the present invention, it has been disclosed that Gal-9 enhances cytotoxicity and NK-like activity (though the activity is at a low level) against Meth-A cells.

Putting these together, it is suggested that NK cells stimulated with BALL-mf are effective against other tumor cells.

Until two weeks passed after inoculation of tumors, the tumor growth is similar among BALL-mf, Daudi-mf and PBS treated mice (Fig. 1(a)). These indicate that BALL-mf contains at least a member selected from factors causing NK activity and Gal-9 production.

The appearance of tumor cells in BALL-mf treated mice is different from that in Daudi-mf treated ones. In BALL-mf treated mice, several pyknotic tumor cells were found which were located extremely close to the fibrous periphery of tumor tissue while in Daudi-mf treated mice no pyknotic cells were found et all (Fig. 3a & Fig. 2c). It is well-known that galectins play an important role in apoptosis. For example, it has been disclosed that galectin 1 induces apoptosis in T cells while galectin 3 inhibits cell death. Recently, it is shown that the overexpression of galectin 7 is perhaps involved in the apoptosis process of UV (sunshine)-induced keratinocytes. For Gal-9, it has been reported that mouse galectin 9 induces apoptosis in thymocytes and activated T lymphocytes.

It has been found herein that the expression of Gal-9 and the induction of apoptosis did not occur even when tumor cells were stimulated with BALL-mf. Gal-9 itself induced apoptosis in the tumor cells. These suggest that BALL-mf does not exert antitumor effect by a direct action on tumor cells but does via the expression and/or release of Gal-9 in T cells and mast cells.

Putting in vitro tissue specimen results and PC immunostaining results together, it is apparent that mast cells, macrophages, granulocytes (in particular, eosinophils) are Gal-9-containing cells. Therefore, mast cell line, MC9, is used for experiments. The expression of galectin 9 was examined for mast cell line, MC9. When MC9 cells were stimulated with BALL-mf, the expression of cell-surface galectin 9 was slightly enhanced at 24 hours post-treatment, but no expression of cytoplasmic galectin 9 was observed to be enhanced.

Example 2

[Purification of Galectin 9-Inducer]

The BALL-1 cell-derived soluble cell membrane fraction (BALL-1 mf), obtained in Example 1, was used as a starting material for further purification steps. When unadsorbed fractions on a lentil lectin column were separated from adsorbed fractions, most of the galectin 9-inducing activity was observed to be contained in the adsorbed fractions. In antitumor activity assay experiments, the antitumor efficacy levels of the adsorbed fraction (Eluate) were observed to be comparable to those of Original (BALL-mf). The infiltration levels of eosinophils and mast cells for the adsorbed fraction were similar to those for Original.

RNA samples were collected from peripheral blood mononuclear cells stimulated with fraction products obtained by IEF fractionation of the lectin column-adsorbed fractions with the Rotofor® system, and examined by RT-PCR for the expression of galectin 9. In RT-PCR, it was ascertained that the expression of galectin 9 was apparently enhanced in fractions, F-1, F-2, and F-4, resulting from the aforementioned IEF fractionation. These fractions resulting from the aforementioned IEF fractionation were examined for their antitumor activity. As a result, it was ascertained that the intense antitumor activity was induced by fractions, F-2 and F-3. The efficacy of F-1 and F-4 is similar to that of PBS, or with increased tumor cell growth. The antitumor activity was observed to be owned by galectin 9-inducers contained in F-2 and F-3. The infiltration of eosinophils and mast cells was observed with tissue staining for the fractions, F-2 and F-3.

Example 3

[BALL-1 Cell Solubilization]

BALL-1 cells, cultured in 10% FCS-containing RPMI1640 medium, were used as starting sources. Harvested BALL-1 cells were resuspended in 1 mM phenylmethylsulfonyl fluoride (PMSF)-PBS ($1 \times 10^9$ cell/5 mL). Four freeze-thawing cycles were carried out with liquid nitrogen and water at room temperature (freeze-thawingx4 cycles). Next, sonication (output=4, duty cycle%=50, on ice, for 4 min (substantial period: about 2 min)) was conducted. The sonication was operated in such a manner that samples were sonicated for 2 min, next rested, and then resonicated for 2 min. The disrupted products were centrifuged. The centrifugation was performed at 100,000 G for 1 hr at 4°C.

Pellets from the centrifugation step were refloated in a solution consisting of 50 mM Tris-HCl (pH 8.2), 1 mM EDTA and 1% CHAPS at a volume equivalent to the aforementioned volume at which the BALL-1 cells were resuspended in 1 mM PMSF-PBS, and homogenized. The homogenization was conducted with a 10 mL or 20 mL Teflon® glass homogenizer on ice for several minutes until pellets disappeared completely. The resultant products were centrifuged. The centrifugation was performed at 20,000 G for 30 min at 4°C.

Supernatants were collected (Sup(MF)). The optical density (OD) was measured. A blank run used is a solution consisting of 50 mM Tris-HCl (pH 8.2), 1 mM EDTA, and 1% CHAPS.

[Column Chromatographic Purification]

(1) The resultant MF from the foregoing step was applied to column chromatography on ligand, concanavalin A (Con A)-coupled carriers.

Con A Sepharose® beads were admixed with MF at a ratio of 1:1 (bead:MF), and incubated at 4°C with rotation by O/N. When MF was concentrated, the admixture was done after diluting the MF 2-fold with PBS(-). Next, this was applied to a column.

Column conditions:

Poly-Prep® chromatography column (BIO-RAD 731-1550)
Column volume, 1.6 to 2 mL
Flow down = Elu: freely flow down at 22G, Ft, wash: gravity flow without a syringe
Beads = Con A Sepharose® beads (Pharmacia)
(Pretreatment of beads: The beads were washed with $H_2O$, next centrifuged at 1,000 rpm for 1 min at 4°C, and then equilibrated)
Equilibration buffer = TBS containing 1 mM $CaCl_2$, and 0.1% CHAPS
(10 or more bed volumes of equilibration buffer were passed through)

WASH = equilibration buffer
Elute = Fr. 1 to 2: 0.1 M borate buffer (pH 6.5) (Borate)
Fr. 3 to : 0.2 M boric acid, 0.15 M NaCl
Preservation = PBS containing 0.02% NaN$_3$, kept at 4°C

Eluted Ft was collected. For WASH, the flowthrough volume of equilibration buffer is equal to the volume of applied beads. An elution buffer was applied, then a stopper was put, and the column was maintained at room temperature for 20 min. Each 1 mL fraction was collected at 5 minute intervals, and the OD (280 nm) was checked.

(2) The resultant chromatographically treated Con A affinity column fractions were applied to anion exchange column chromatography.

The resultant BALL-1 mf Con A-fraction (9 mL; eluted with 0.1 M borate-NaOH (pH 6.5)), from the foregoing step, was applied to anion exchange column chromatography on RESOURCE® Q (1 mL, Amersham Biosciences).

Buffer: A, 10 mM Tris-HCl (pH 7.5), 0.03% CHAPS
B, 10 mM Tris-HCl (pH 7.5), 1 M NaCl, 0.03% CHAPS
Gradient: %B=0 → 100 in 25 min.
Flow rate: 1 mL/min.
Fraction volume: 1 mL
Monitor: UV (A$_{280}$ nm) 0-0.05
Conductivity, 0-100 mS

Samples were concentrated (x10) with StrataClean® Resin (Stratagene, CA, USA). The concentrated samples were applied to SDS-PAGE: 12% gel, and stained with SYPRO® Orange (Molecular Probes, Inc., USA).

(3) The resultant chromatographically treated RESOURCE® Q column fractions were applied to hydroxyapatite column chromatography.

The resultant BALL-1 mf RESOURCE® Q-fraction (fraction Nos. 14 to 17), from the foregoing step, was applied to hydroxyapatite column chromatography on CHT2-I (Bio-Rad).

Column: CHT2-I (Bio-Rad), 2 mL
Buffer: A, 10 mM Na-Pi (pH 6.8), 0.03% CHAPS, 0.05% NaN$_3$
B, 500 mM Na-Pi (pH 6.8), 0.03% CHAPS, 0.05% NaN$_3$
Gradient: %B=0 → 80 in 30 min.
Flow rate: 1 mL/min.
Fraction volume: 1 mL
Monitor: UV (A$_{280}$ nm) 0-0.02
Conductivity, 0-50 mS

Samples were concentrated (x40) with StrataClean® Resin (Stratagene, CA, USA). The concentrated samples were applied to SDS-PAGE: 12% gel, and stained with SYPRO® Orange (Molecular Probes, Inc., USA).

(4) The resultant chromatographically treated RESOURCE® Q column fraction D was applied to hydroxyapatite column chromatography.

The resultant BALL-1 mf RESOURCE® Q-fraction D, from the foregoing step, was applied to hydroxyapatite column chromatography on CHT2-I (Bio-Rad).

Column: CHT2-I (Bio-Rad)
Buffer: A, 10 mM Na-Pi (pH 6.8), 0.03% CHAPS
Buffer: B, 500 mM Na-Pi (pH 6.8), 0.03% CHAPS
Gradient: %B 0 → 180 in 30 min.
Flow rate: 1 mL/min.
Fraction volume: 1 mL
Monitor: UV (A$_{280\ nm}$) 0-0.02
Conductivity, 0-50 mS

Samples were concentrated (×40) with StrataClean® Resin (Stratagene, CA, USA). The concentrated samples were applied to SDS-PAGE: 12% gel, and stained with SYPRO® Orange (Molecular Probes, Inc., USA).

[Biological Activities of Chromatographically Purified BALL-mf Fractions]

The solubilized tumor cell line cell-membrane fraction was subjected to purification steps on a lentil lectin column followed by IEF. As a result, a galectin 9-inducer candidate was narrowed down to the resulting 4 fractions with antitumor activity; however, since next purification steps were expected to require a lot of laborious work and time because the amount of recovered proteins was little, extracting and purifying methods were anew searched and examined. Thus, a concanavalin A (Con A) column with not only similar binding specificity to that of the lentil lectin column but also more binding capacity was used for further purification steps.

The soluble cell membrane fractions were loaded on a Con A column to give adsorbed and unadsorbed fractions. BALL-mf was fractionated on a Con A column. As a result, unadsorbed fractions from the Con A column were separated from Con A column-adsorbed fractions. Electrophoresis (SDS-PAGE) of the resultant fractions were observed to give distinct protein bands (Fig. 16). When each fraction was subcutaneously injected into Meth-A bearing mice, the adsorbed fraction (A) was observed to have potent antitumor activity (Fig. 17). The unadsorbed fraction (B) suppressed tumor growth as compared to PBS, but failed to eradicate tumors (Table 1). When tissue specimens in the periphery of sites injected with the adsorbed fraction were examined with an optical microscope, pyknotic cells were found among cells in the surface layer of tumor, suggesting the possibility of apoptosis (Fig. 18). In contrast, no cytotoxicity was found against normal cells.

Next, purification on an anion exchange column (RESOURCE Q) was performed. Eluate fractions were classified into 7 fractions (designated in elution order: A, B, C, D, E, F, G) according to electrophoretic patterns of respective fractions, and examined for their antitumor activity (Fig. 19). As a result, the fraction D was observed to have most intense antitumor activity. Further, when the antitumor activity of fraction D was examined by varying its concentration (dilution ratio: 1,200, 6,000, and 30,000-fold), the antitumor activity was observed to be concentration-dependent (Fig. 20). With regard to the cytotoxicity, its cancer cell-specificity can be examined through experiments using prepared tissue specimens.

Table 1 Antitumor Effects of Con A Column-Purified Fractions

| Con A column | Eradicated/Transplanted (Animals) |
|---|---|
| Adsorbed Fr. | 15/5 |
| Unadsorbed Fr. | 2/18 |
| PBS | 3/17 |

After purification on anion exchange column, RESOURCE® Q, the chromatographically treated RESOURCE® Q column fraction D was applied to chromatography on a hydroxyapatite column to give fractions, A to E (Fig. 21). The SDS-PAGE results of respective hydroxyapatite column fractions are also shown in Fig. 21. When antitumor activity was examined in the same fashion as aforementioned, the hydroxyapatite column CHT2-I fraction D was observed to have most potent antitumor activity as compared to other fractions (Fig. 22). The SDS-PAGE results of said CHT2-I fraction D are also shown in Fig. 22.

In Fig. 1, each symbol stands for the following:

In Fig. 1(a),

    • : Tumor weight in BALL-mf treated animals.
    ■ : Tumor weight in Daudi-mf treated animals.
    ○ : Tumor weight in PBS treated animals.

In Fig. 1(b),

    • : Number of BALL-mf treated animals wherein tumor rejection was induced.
    ■: Number of Daudi-mf treated animals wherein tumor rejection was induced.
    ○: Number of PBS treated animals wherein tumor rejection was induced.

INDUSTRIAL APPLICABILITY

The galectin 9-inducing factors (galectin 9-inducers) are successfully identified and purified herein, thereby leading to applications of said purified galectin 9-inducers in order to develop pharmaceutical products and to accelerate research and development of physiological phenomena and biological actions associated with galectin 9. In particular, galectin 9-inducers are contained in a solubilized cell membrane fraction, a concanavalin A-adsorbed fraction from said fraction, and a concentrated active fraction derived by fractionation with a Resource Q® ion exchange column, a hydroxyapatite column, etc. Administration of said inducer leads to occurrence of biological activities including antitumor activity and the activity of increasing or strengthening natural killer activity and others. Therefore, it will be possible to develop assay reagents, pharmaceutical products, assays and the like based on adaptations of said galectin 9-inducing activity.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

<Sequence Listing Free Text>

SEQ ID NO: 1, Oligonucleotide to act as a primer for PCR
SEQ ID NO: 2, Oligonucleotide to act as a primer for PCR
SEQ ID NO: 3, Oligonucleotide to act as a primer for PCR
SEQ ID NO: 4, Oligonucleotide to act as a primer for PCR
SEQ ID NO: 5, Oligonucleotide to act as a primer for PCR
SEQ ID NO: 6, Oligonucleotide to act as a primer for PCR

**Claims**

1. A human galectin 9-inducer which has an identifiable biological activity existing in a soluble cell membrane fraction derived by solubilization of insoluble cell lysates of human lymphoid B cell lines, BALL-1 cells (B lymphoma cells), wherein the biological activity of said galectin 9-inducer can be identified by at least a property selected from the group consisting of:

   (1) galectin 9-inducing activity,
   (2) ability to incite inhibition or suppression of tumor cell growth and/or tumor rejection in an in vivo test wherein Meth-A sarcoma cells are used as tumor cells to be targeted,
   (3) antitumor activity,
   (4) ability to induce the natural killer activity of peripheral blood mononuclear cells in an in vitro test,
   (5) up-regulation of galectin 9 mRNA expression in a test wherein peripheral blood mononuclear cells are used,
   (6) significant elevation in the cytoplasmic expression of galectin 9 proteins in a test wherein peripheral blood mononuclear cells are used,
   (7) the formation of recognizable granulation tissue composed of eosinophils and mononuclear cells, accompanied with few neutrophils, at a site injected with said galectin 9-inducer when histopathologically examined,
   (8) the induction of a large number of observable mast cells at connective tissues over or underneath the cutaneous muscle layer of said galectin 9 inducer-injected site,
   (9) the induction of observable regions with infiltrated inflammatory cells (predominant eosinophils and a few mast cells), at the periphery of tumors or being located within tumor tissues, when the tumors or the peripheral areas of tumors are histopathologically examined,
   (10) the formation of observable tumor cells showing pyknotic changes when the tumors or the peripheral areas of tumors are histopathologically examined, and
   (11) the occurrence of observable metachromatic mast cell accumulation in regions at the periphery of tumors or within tumor tissues when the tumors or the peripheral areas of tumors are histopathologically examined.

2. The galectin 9-inducer according to claim 1, wherein the starting cells used as sources are radiated lymphoid B cell lines BALL-1 cells.

3. The galectin 9-inducer according to claim 1 or 2, which exists in a soluble cell membrane fraction derived by solubilization including homogenization of BALL-1 cells with a detergent in the presence of a protease inhibitor.

4. The galectin 9-inducer according to any of claims 1 to 3, which can be purified and/or concentrated from said B lymphoma cell-derived soluble cell membrane fraction by a treatment selected from the group consisting of concanavalin A column chromatography, ion exchange column chromatography, hydroxyapatite column chromatography, and other column chromatographic techniques.

5. A galectin 9-inducing reagent for intracellular induction of galectin 9, which comprises an effective amount of the galectin 9-inducer according to any of claims 1 to 4.

6. A method for intracellular induction of galectin 9, which comprises contacting a cell with an effective amount of the galectin 9-inducer according to any of claims 1 to 4.

7. A pharmaceutical drug which comprises an effective amount of the galectin 9-inducer according to any of claims 1 to 4.

8. The pharmaceutical drug according to claim 7, which is selected from antineoplastic drugs, anti-inflammatory drugs, antiallergic drugs, immunosuppressants, drugs for auto-immune diseases, and adrenal cortical steroid hormone alternatives.

FIG. 1

FIG. 2

FIG. 3

FIG. 4

FIG. 5

FIG. 6

FIG. 7

FIG. 8

PBS 50.5    Antigalectin 9 Adsorption 325.3

Antigalectin 8 Adsorption    Original 559.5

118.5

FIG. 9

Chi-square analysis

PBS          35=29/6
Cont         25=22/3
BALL-mf      30=6/24

                    Rejected

FIG. 10

Daudi-mf⁻ periphery

Daudi-mf⁻ center

BALL-mf⁻ periphery

BALL-mf⁻ center

FIG. 11

FIG. 12 A

FIG. 12 B

FIG. 12 C

FIG. 12 D

FIG. 13

1  2  3  4  5  6  7  8  9  10

⟷    ⟷    ⟷    ⟷

F-1        F-2      F-3        F-4

PI    3~5      5.1~6.0   6.1~7.1   7.2~9.5

FIG. 14

FIG. 15

Con A Column-Unadsorbed Fraction | Con A Column-Adsorbed Fraction

FIG. 16

Antitumor Efficacy of Concanavalin A Column-Purified Fractions

FIG. 17

FIG. 18

Antitumor Efficacy Examination of
Anion Exchange Column (RESOURCE Q®)-Purified Fractions

FIG. 19

Antitumor Efficacy Examination of Fraction D

FIG. 20

# Hydroxyapatite Column Chromatography of RESOURCE Q® Fraction D

Sample: RESOURCE Q, Fraction D
Column: CHT2-I (hydroxyapatite column; Bio-Rad)
Buffer A: 10 mM Na-Pi (pH6.8),
    0.03% CHAPS
Buffer B: 500 mM Na-Pi (pH6.8),
    0.03% CHAPS
Gradient: %B 0→180 in 30 min

FIG. 21

EP 1 619 203 A1

Antitumor Efficacy of CHT2-I Fractions

FIG. 22

EP 1 619 203 A1

| INTERNATIONAL SEARCH REPORT | International application No. |
|---|---|
| | PCT/JP2004/006212 |

**A. CLASSIFICATION OF SUBJECT MATTER**
Int.Cl⁷ C07K14/52, C12N15/09, A61K38/22, A61P5/38, 29/00, 35/00, 37/06, 37/08

According to International Patent Classification (IPC) or to both national classification and IPC

**B. FIELDS SEARCHED**

Minimum documentation searched (classification system followed by classification symbols)
Int.Cl⁷ C07K14/00-16/46, C12N15/00-90, A61K38/22, A61P5/38, 29/00, 35/00, 37/06, 37/08

Documentation searched other than minimum documentation to the extent that such documents are included in the fields searched

Electronic data base consulted during the international search (name of data base and, where practicable, search terms used)
JICST FILE(JOIS), EUROPAT(QUESTEL), MEDLINE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| A | Mitsuomi HIRAJIMA, "Galectin to Ensho", Clinical Immunology, 2002, 38(1), pages 58 to 63 | 1-8 |
| A | Mitsuomi HIRAJIMA "Men'eki Shikkan o Meguru Kisoteki Kenkyu no Shinpo 34 Galectin Kami ga Sozo shita Chie no aru Nori", Igaku no Ayumi Bessatsu (Men'eki Shikkan Zenmen Kaitei Han), 2002, p.170-4 | 1-8 |
| A | Mitsuomi HIRAJIMA "Kosankyu Atarashii Chemokine Ecalectin", Igaku no Ayumi, 2000, 192(1), p.1066-70 | 1-8 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 May, 2004 (21.05.04) | 29 June, 2004 (29.06.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

# EP 1 619 203 A1

## INTERNATIONAL SEARCH REPORT

International application No.

PCT/JP2004/006212

C (Continuation). DOCUMENTS CONSIDERED TO BE RELEVANT

| Category* | Citation of document, with indication, where appropriate, of the relevant passages | Relevant to claim No. |
|---|---|---|
| A | Yumiko KASHIO et al., "Atarashii Kosankyu Soka Inshi toshiteno Ecalectin/galectin-9", Clinical Immunology, 2000, 34(2), p.226-30 | 1-8 |
| A | H. ASAKURA et al., Selective Eosinophil Adhesion to Fibroblast Via IFN-γ-Induced Galectin-9, The Journal of Immunology, 2002, 169(10), p.5912-8 | 1-8 |
| A | O. TURECI et al., Molecular Definition of a Novel Human Galection Which Is Immunogenic in Patients with Hodgkin's Disease, The Journal of Biological Chemistry, 1997, 272(10), p. 6416-22 | 1-8 |
| A | R. MATSUMOTO et al., Human Ecalectin, a Variant of Human Galectin-9, Is a Novel Eosinophil Chemoattractant Produced by T Lymphocytes, The Journal of Biological Chemistry, 1998, 273(27), p.16976-84 | 1-8 |
| A | H. YOSHIDA et al., Interleukin-1β stimulates galectin-9 expression in human astrocytes, NEUROCHEMISTRY, 2001, 12(17), p.3755-8 | 1-8 |
| P,A | JP 2003-189874 A (Galpharma Co., Ltd.), 08 July, 2003 (08.07.03), (Family: none) | 1-8 |
| A | WO 1999/010490 A1 (Sagami Chemical Research Center), 04 March, 1999 (04.03.99), & JP 2001-522581 A  & US 2004/068104 A1 & EP 1005548 A1 | 1-8 |
| A | WO 1998/015624 A1 (HUMAN GENOME SCI. INC.), 16 April, 1998 (16.04.98), & JP 2001-501831 A  & US 6027916 A & EP 1012266 A1 | 1-8 |

Form PCT/ISA/210 (continuation of second sheet) (January 2004)

## EP 1 619 203 A1

<table>
<tr>
<td>INTERNATIONAL SEARCH REPORT</td>
<td>International application No.<br><br>PCT/JP2004/006212</td>
</tr>
</table>

**Box No. I    Nucleotide and/or amino acid sequence(s) (Continuation of item1.b of the first sheet)**

1.  With regard to any nucleotide and/or amino acid sequence disclosed in the international application and necessary to the claimed invention, the international search was carried out on the basis of:

    a.   type of material

        [X]  a sequence listing

        [ ]  table(s) related to the sequence listing

    b.   format of material

        [ ]  in written format

        [X]  in computer readable form

    c.   time of filing/furnishing

        [ ]  contained in the international application as filed

        [X]  filed together with the international application in computer readable form

        [ ]  furnished subsequently to this Authority for the purposes of search

2.  [X]   In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3.  Additional comments:

Form PCT/ISA/210 (continuation of first sheet (1)) (January 2004)

**INTERNATIONAL SEARCH REPORT**

International application No.

PCT/JP2004/006212

Box No. II    Observations where certain claims were found unsearchable (Continuation of item 2 of first sheet)

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.: parts of 1-8
   because they relate to parts of the international application that do not comply with the prescribed requirements to such an extent that no meaningful international search can be carried out, specifically:
   (See extra sheet.)

3. ☐ Claims Nos.:
   because they are dependent claims and are not drafted in accordance with the second and third sentences of Rule 6.4(a).

Box No. III    Observations where unity of invention is lacking (Continuation of item 3 of first sheet)

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest.

                          ☐ No protest accompanied the payment of additional search fees.

Form PCT/ISA/210 (continuation of first sheet (2)) (January 2004)

| INTERNATIONAL SEARCH REPORT | International application No. |
| --- | --- |
| | PCT/JP2004/006212 |

Continuation of Box No.II-2 of continuation of first sheet(2)

Claims 1 to 8

It is unknown what substance the "galectin 9-inducing factor" in the above claims is. Thus, it does not appear that these claims are clearly described.

Concerning the inventions according to the above claims, it is not proved that an activity of inducing galectin 9, an antitumor activity, etc. are exerted, excluding the solubilized cell membrane fraction obtained from B cell lymphoma-origin cell line BALL-1 cells prepared by a method as described in EXAMPLES. It is therefore unknown whether or not fractions obtained by other methods would show an activity of inducing galectin 9, an antitumor activity, etc. Therefore, these inventions are neither fully supported by the description nor disclosed therein in a manner sufficiently clear and complete for a person skilled in the art to carry out the inventions.

No search was made on the inventions which are neither clearly described in the claims nor fully supported by the claimed description, or the inventions which are not disclosed in the description in a manner sufficiently clear and complete for a person skilled in the art to carry out the inventions, that is, the inventions other than the invention relating to the solubilized cell membrane fraction obtained from B cell lymphoma-origin cell line BALL-1 cells prepared by a method as described in EXAMPLES.

Form PCT/ISA/210 (extra sheet) (January 2004)